# EUROPEAN PATENT SPECIFICATION

(11) **EP 2 966 448 B1**
(45) Date of publication and mention of the grant of the patent: **05.03.2025**
(21) Application number: 14749402.5
(22) Date of filing: 10.02.2014
(51) Int. Cl.: G01N 33/574, A61K 31/445, A61K 35/00, A61P 35/00, A61K 31/496, A61K 31/5377, C12Q 1/68, G01N 33/566

(54) **METHOD FOR THE PREDICTION OR PROGNOSIS OF THE RESPONSE OF A HUMAN SUBJECT SUFFERING FROM CANCER TO TREATMENT WITH AN ANTAGONIST OF THE NK1 RECEPTOR**
VERFAHREN ZUR VORHERSAGE ODER PROGNOSE DER REAKTION EINES KREBSPATIENTEN AUF EINE BEHANDLUNG MIT EINEM ANTAGONISTEN DES NK1-REZEPTORS
MÉTHODE DE PRÉDICTION OU DE PRONOSTIC DE LA RÉPONSE D'UN SUJET HUMAIN ATTEINT D'UN CANCER AU TRAITEMENT AVEC UN ANTAGONISTE DU RÉCEPTEUR NK1

(30) Priority: 11.02.2013 ES 201330174
(43) Date of publication of application: 13.01.2016
(73) Proprietor: Servicio Andaluz de Salud, 41071 Sevilla (ES)
(72) Inventor: SALINAS MARTÍN, Manuel Vicente, E-41071 Sevilla (ES)
(74) Representative: J A Kemp LLP
(86) International application number: PCT/ES2014/070090
(87) International publication number: WO 2014/122353

(56) References cited:
- EP-A1- 1 803 456
- EP-A1- 2 604 276
- EP-A2- 0 773 026
- EP-A2- 0 773 026
- WO-A1-2005/077366
- WO-A1-2009/124756
- J. BUSSOLINI: "What Is a Dispositive?", FOUCAULT STUDIES, no. 10, 2010, New York NY USA, pages 85 - 107, XP055297160
- MUNOZ MIGUEL ET AL.: "The substance P/neurokinin-1 receptor system in lung cancer: Focus on the antitumor action of neurokinin-1 receptor antagonists''.", PEPTIDES, vol. 38, no. 2, December 2012 (2012-12-01), NEW YORK, pages 318 - 325, XP055219145

## Description

### TECHNICAL FIELD

This invention is within the field of medicine, pharmacy and molecular biology, and more concretely, in the field of oncology. Specifically it is related to a method for the prediction of the response of a human subject suffering from cancer to the treatment with an antagonist of the NK1 receptor, that allows the subjects to be classified into responders and no responders. The invention also relates to the use of the expression level of the NK1 receptor as an indicator of treatment response.

### STATE OF THE ART

NK1 receptors (Neuropeptide receptors of Substance P and tachykinins) are widely distributed in the body cells. Currently, multiple biological processes are known in which NK1 receptors are involved in their regulation. NK1 receptors are receptors of type of G protein-coupled receptors.

Like other receptors, NK1 receptors have their own natural agonists. The most selective of these is Substance P. Substance P (SP) is an undecapeptide of natural origin, which belongs into the family of the tachykinins, is produced by mammalians, and its sequence was described by Veber et al., (US 4,680,283). The tachykinin family also includes other peptides such as Neurokinin A, Neurokinin B, Neuropeptide K, Neuropeptide Gamma, and Hemokinin I, among others. The participation of SP and the other tachykinins in the etiopathogenesis of several diseases has been widely referred in scientific research. In this sense, the action of tachykinins has been connected with the etiopathogenesis of diseases of the human nervous system such as Alzheimer's disease, multiple sclerosis, Parkinson's disease, anxiety, and depression (Barker R. et al., 1996; Kramer MS, et al., 1998). The participation of tachykinins has also been verified in the ethyopathogenesis of several diseases having an inflammatory element, such as rheumatoid arthritis, asthma, allergic rhinitis, intestinal inflammatory diseases such as ulcerous colitis, and Crohn's disease (Maggi CA, et al., 1993).

In this sense, the pharmaceutical industry has developed non-peptide NK1 receptor antagonists as medicines for the treatment of several disorders of the central nervous system, such as depression, psychosis, and anxiety (WO 95/16679, WO 95/18124, WO 95/23798 y WO 01/77100). It has been described that the use of selective NK1 receptor antagonists is useful in the treatment of nauseas and vomiting induced by antineoplastic chemotherapeutic agents, as well as in the treatment of several forms of urinary incontinence (Quartara L. et al., 1998; Doi T. et al., 1999).

In a study published in 2003 (Giardina G, et al., 2003) and in another one published in 2010 (Huang SC, et al., 2010) a revision of the most recent patents about NK1 receptor antagonists is shown. The molecules of the most important world manufacturers are described with indications concerning their potential possible applications, including mainly: antidepressive, antiinflammatory, anxiolytic, antiemetic, treatment of ulcerative colitis, and other.

It has been published the use of several SP antagonists to inhibit proliferation of lung cell carcinoma (Orosz A, et al., 1995; Bunn PA Jr. et al., 1994).

It is known that the astrocytes of the central nervous system express functional receptors for several neurotransmitters, including the NK1 receptors (Palma C. et al., 2000). In brain tumors, malignant glial cells derived from astrocytes trigger, under the tachykinin action and mediated by mediation of NK1 receptors the secretion of mediators increasing their proliferation rate. Consequently, selective NK1 antagonists can be very useful as therapeutic agents for the treatment of malignant gliomas.

Patent EP 773026 (Pfizer) makes reference to the use of non-peptide NK1 receptor antagonists for the treatment of cancer in mammals. Particularly in the treatment of small cell pulmonary carcinomas, APUDOMAS (tumors of enterochromaffin cells, which are predominantly located in the mucosa of the digestive track. The letters stand for "Amine Precursors Uptake and Decarboxylation").

On the other hand, patent WO 2001001922 describes the use of NK1 receptors for the treatment of adenocarcinomas, and very specifically, for prostatic carcinomas.

Several studies with specific antagonist of neurokinin receptors such as CP-96341-1 (Pfizer), MEN 11467, SR 48968 (Sanofi) and MEN 11420 (Nepadutant) have shown their efficacy in blocking cellular proliferation (Singh D et al., 2000; y Bigioni M. et al., 2005).

It has been described that non-peptide NK1 receptor antagonists induce apoptosis (cell death) in cancer cells in several tumors such as stomach carcinoma, colon carcinoma (Rosso M, et al., 2008), melanoma (Muñoz M, et al., 2010), or lung carcinoma (Muñoz M, et al., 2012).

Patent ES 2246687 claims the use of non-peptide NK1 receptor antagonists and of SP for the manufacture of a pharmaceutical composition for the induction of apoptosis in mammals cancer tumor cells. The Spanish patent application P201101311 (PCT/ES2012/070865) claims the use of modifying agents from the peritumor microenvironment, concretely the NK1 receptor antagonists, for the treatment of cancer. On the other hand, the patent application WO2012020162 describes the use of antibodies, or fragment thereof, against NK1, NK2 and/or NK3 receptors as useful in the treatment of cancer due to the production of apoptosis of tumor cells.

Currently, several medicaments directed to antagonize the effects of given membrane receptors aiming to treat cancer are commonly used in clinical practice. The most representative examples are the use of monoclonal antibodies against Her2 receptor to treat breast and stomach cancer, the use of medicines inhibiting Tyrosine Kinase to treat lung cancer, and the use of monoclonal antibodies directed against EGFR receptors (Receptor for the Epidermal Growth Factor) to treat colon cancer, among others. In all these cases the existence of predictive biological markers for response to such treatments is known. For instance, in the case of breast cancer it is known that, in humans, the monoclonal antibody directed against Her-2 receptor is effective only if there is an overexpression of this receptor, which is observed by means of an immunohistochemical study or an amplification of the gene. This amplification can be observed by marking this gene with specific probes which can be visualized using "in situ" hybridization methodologies. In the case of lung cancer treatment with tyrosine kinase inhibitors medicines, it is known that these medicines are only effective if there is a mutation of the gene encoding for the EGFR receptor which conditions the alteration of this gene's structure which, likewise, conditions that this receptor becomes "constitutively" and permanently activated. If such a mutation does not exist in this gene, the above referred medicines are not effective to treat lung cancer. In the case of colon cancer it is known that treatment with monoclonal antibodies directed against EGFR receptor is effective only if there is no mutation at the level of the K-ras gene. If there is a mutation at the level of this gene, it conditions a change at the level of the protein encoded by this gene, so that it becomes constitutively activated, such antibodies are not effective in the treatment of these cancerous colon tumors.

Scientific research has described how some tumor cells can have an NK1 receptor truncated form (Gillespie E, et al. 2011).

Therefore, in conclusion, the following facts are currently known in the state of the art:
1. That NK1 receptors are widely spread in human organisms.
2. That tachykinins, and more concretely the SP, act on NK1 receptors.
3. That the use of non-peptide NK1 receptor antagonists has shown effects on tumor cells, which lead to their cellular death by apoptosis (ES 2246687).
4. That the use of agents modifying of tumor microenvironment, concretely the NK1 receptor antagonists, is useful in the treatment of cancer tumors (PCT/ES2012/070865).
5. That the use of antibodies, or fragment thereof directed against NK1 receptors, is useful to treat cancer, due to their ability to provoke the apoptosis of tumor cells (WO2012020162).
6. That some tumor cells can have the truncated form of an NK1 receptor.
7. That there are predictive markers of response to several treatments seeking to treat cancer by means of the modification of the functioning of several membrane receptors of tumor cells, others than NK1 receptors.

It is known in the state of the art that the MAP Kinase pathway, specifically by means of its efferent pathways such as, for instance, ERK or MEK, can modulate cell proliferation by means of several remarkable efferent pathways, such as Fos/Jun or P90rsk, among others. On the other hand, it is also known in the state of the art that the activation of the PI3 Kinases pathway leads to the increase of one of its final afferent pathways, such as AKT. Therefore, some tumors use the MAP Kinases and the PI3 Kinases pathways, which benefit their competitiveness concerning their capability to proliferate. These tumors present high quantities of proteins ERK, MEK, and AKT, which are characteristic of these pathways (MAP Kinases and PI3 Kinases).

RAS protein is a key element in the MAP Kinases and PI3 Kinase pathways. The Ras protein occupies a key location downstream in the signalling pathways of the MAP Kinases and PI3 Kinases. The mutation of the K-ras gene conditions the expression of a protein "constitutively activated" that maintains such pathways permanently activated, independently of the state of the receptor and of its afferent upstream, therefore sending a signal to activate these pathways continuously, which induces a permanent stimulation of cellular proliferation.

However, the known prior art, including using NK1 receptor antagonists (non-peptide antagonists or antibodies -or fragments thereof-) for the production of death and/or apoptosis of tumor cells (either by direct action on these tumor cells or by modification of their microenvironment), it is presently unknown which groups of patients could respond better to the treatment with NK1 receptor antagonists. To know this would allow us to establish the most adequate doses of NK1 receptor antagonists, and its use in combination with other anti-cancer treatments (for instance, chemotherapeutics or specific blockers of other molecular pathways relevant to certain types of tumors) or treatments specific to support oncological patients, to achieve a more effective and efficient result.

Munoz et al. (Peptides, vol. 38, no.2, December 2012, pages 318-325), discusses the involvement of substance P/NK1 receptor system in human lung cancer cell lines.

EP 0 773 026 A2 discusses NK-1 receptor antagonists (e.g. Substance P receptor antagonists) for the manufacture of a medicament for the treatment of cancer in a mammal.

EP 1 803 456 A1 discusses of substance P antagonists and, in particular, the use of non-peptidic NK1 receptor antagonists for the treatment of cancer.

EP 2 604 276 A1 discusses the use of an antibody or a fragment thereof specific against NK1, NK2 and/or NK3 receptors of cells, for the manufacture of a medicament for the therapeutic management of human cancer.

WO 2009/124756 A1 discusses the use of heterocyclic compounds such as aprepitant for the treatment of cancer.

WO 2005/077366 A1 discusses the use of substance P antagonists and, in particular, the use of non-peptidic NK1 receptor antagonists for the treatment of cancer.

J. BUSSOLINI: "What Is a Dispositive?", FOUCAULT STUDIES, no. 10, 2010, pages 85-107, discusses the translations of French and Italian terms having meanings similar to "apparatus" in English.

### DESCRIPTION OF THE INVENTION

### Brief description of the invention

A first object of the present invention is the use of an *in vitro* method to predict the response of a human subject suffering from cancer to a treatment of said disease with an antagonist of the NK1 receptor, wherein said method comprises:
- detecting the expression level of the NK1 receptor in a sample of tumor cells isolated from the subject;
- comparing the expression level to a reference value; and
- predicting a positive response to the treatment if the expression level is increased relative to the reference value,
wherein the reference value is the average expression level in a population of non-respondents.

Preferably, the expression levels of the NK1 receptor or of the mRNA that encodes for the NK1 receptor, are detected in a sample that includes tumor cells of a human subject with cancer. In another preferred embodiment, the presence or absence of mutations in the K-ras gene is determined in the tumor cells of an isolated sample. In another preferred embodiment, the presence or absence of the ERK protein is determined in the tumor cells of an isolated sample. More preferably, the presence or absence of the AKT protein is determined in the tumor cells of the isolated sample. Even more preferably, the presence or absence of the truncated form of the NK1 receptor is determined in the tumor cells of the isolated sample. Even much more preferably, it is determined if the tumor cells of the isolated sample present a constitutively activated form of the NK1 receptor.

A second object of this invention refers to the use of the expression level of the NK1 receptor in a sample of tumor cells as an indicator in an *in vitro* method to predict if a human suffering from cancer will have a positive response to treatment with a pharmaceutical composition comprising an antagonist of the NK1 receptor.

Preferably the antagonist of the NK1 receptor is a non-peptidic or peptidic antagonist. More preferably, the non-peptidic antagonist is selected from Aprepitant, Vestipitant, Casopitant, Vofopitant, Ezlopitant, Lanepitant, LY-686017, L-733,060, L-732,138, L-703,606, WIN 62,577, CP-122721, TAK637, y R673, CP-I00263, WIN 51708, CP-96345, L-760735, L-758298, L-741671, 10 L-742694, CP-99994, T-2328, or combinations thereof; and the peptidic antagonist is selected from antibodies or fragments of thereof directed against the NK1 receptor.

### Detailed description of the invention

The present invention refers to the use of a marker that is selected among:
a) the level of expression of NK1 receptor, or the mRNA that encodes it, and/or
b) the presence of mutation in the K-ras gene, and/or
c) the level of expression of ERK proteins, preferably ERK1 and ERK2, or the mRNA encoding the same and/or
d) the level of expression of MEK proteins, preferably MEK1 and MEK2, or the mRNA encoding the same and/or
e) the level of expression of the AKT protein, preferably AKT1 and AKT2, or the mRNA encoding the same, and/or
f) the presence or not of the truncated form of the NK1 receptor, or the mRNA encoding the same, and/or
g) the presence of the NK1 receptor constitutively activated,
or any of the previous combinations, to predict the response of a human subject to the treatment with a NK1 receptor antagonist. Preferably, the markers are used simultaneously.

### Method to predict or prognosticate the response of a human subject having cancer to the treatment with an antagonist of the NK1 receptor

In the present invention it is shown that tumors presenting an overexpression of the NK1 receptor or an amplification of the NK1 gene, present a better (more effective) response to the treatment with antagonists of the NK1 receptor.

Therefore, a first embodiments of this invention is referred to an *in vitro* method, from now on first method of the invention, to predict or prognosticate the response of a human subject to the treatment with an antagonist of NK1 receptor, in which the subject suffers from cancer, which includes using, as an indicator, the expression levels of the NK1 receptor.

In a preferred embodiment of this aspect of the invention, it is used as indicator, the expression of the NK1 receptor, or the mRNA encoding the same.

The first method of the invention comprises:
a. Obtaining an isolated sample comprising tumor cells of a human subject suffering from cancer, and
b. Detecting the expression levels of the NK1 receptor or the mRNA encoding the NK1 receptor, in the sample obtained in step (a).

Preferably, the result of the first method of the invention is indicative of a positive response (most effective response to the treatment) if the expression levels of NK1 receptor or the mRNA encoding the NK1 receptor are overexpressed in comparison to a sample or reference value and/or a control that expresses levels indicating a lack of response.

More preferably, the overexpression is defined as a level of expression of the NK1 receptor, or the mRNA encoding the NK1 receptor, increased in more than 20% of the cells in the isolated sample obtained in step (a). Even more preferably, the expression level of the NK1 receptor, or the mRNA encoding the NK1 receptor, is increased in more than 30%, 40%, 50%, 60%, 70%, 80%, and even much more preferably, in more than 90% of the cells in the isolated sample obtained in step (a).

In an embodiment, the response is a response to the reduction of the tumor mass. In an alternative embodiment, the response is an improvement or absence of deterioration in the state of the tumor.

In other embodiment, the response is a clinical result, such as the survival without progression or the global survival.

The subjects whose response is predicted are human subjects suffering from cancer. The terms "human subject", "subject", and "patient" are used therefore indistinctively in this specification.

As used herein, the NK1 receptor (also called neurokinin 1 receptor, SPR; NK1R; or TAC1R) is presented as a receptor coupled to a G-protein (GPCR, from English: *G protein-coupled receptors*). This receptor belongs to the G protein-coupled receptors family, with seven transmembrane domains, encoded by the TACR1 gene that in human is found in chromosome 2. This gene belongs to the tachykinin receptor family. These tachykinin receptors are characterized by their interaction with G proteins and they have seven hydrophobic transmembrane regions. This gene encodes for the receptor of substance P tachykinin, also known as neurokinin 1. The encoded protein is also involved in the mediation of the metabolism of the phosphatidylinositol of substance P.

As used herein, TACR1 is defined also by a sequence of nucleotides or polynucleotide, which constitutes the encoding sequence of the protein included in the SEQ ID NO: 1 (GeneBank NP_001049.1), and which would include several variations stemming from:
a) nucleic acid molecules that encode a polypeptide, which includes the amino acid sequence SEQ ID NO: 1,
b) nucleic acid molecules whose complementary chain hybridizes with the polynucleotide sequence of a).
c) nucleic acid molecules whose sequence differs from a) and/or b) due to the degeneration of the genetic code,
d) nucleic acid molecules which encode a polypeptide that includes the amino acid sequence with an identity of at least 80%, 90%, 95%, 98%, or 99%, with the SEQ ID NO: 1, and in which the polypeptide encoded by such nucleic acids has the activity and the structural characteristics of the NK1 protein. Among such molecules of nucleic acid the one included in the sequence SEQ ID NO: 2 is to be found.

As used herein, ERK (*Extracellular-signal-regulated kinases*) is presented as a type of protein that belongs to the molecular pathway of MAP Kinases and which participates in multiple important cellular functions of relevance in the physiopathology of cancer. The MAP kinase (ERK) is a type of serine threonine kinase which can translocate to the nucleus to, once there, regulate the transcription by modifying the activity of proteins (including transcription factors), thusly modulating the expression of different genes. In the context of this invention, ERK1 (also called Mitogen-activated protein kinase 3 or MAPK3) is defined as well by a nucleotides or polynucleotides sequence, which constitute the coding sequence of the protein included in SEQ ID NO: 3 (GeneBank M84490.1), and which would include diverse variants coming from:
a) nucleic acid molecules that encode a polypeptide which includes the amino acid sequence of SEQ ID NO: 3,
b) nucleic acid molecules whose complementary chain hybridizes with the polynucleotide sequence of a)
c) nucleic acid molecules whose sequence differs from a) and/or b) due to the degeneration of the genetic code,
d) nucleic acid molecules which encode a polypeptide that includes the amino acid sequence with an identity of at least 80%, 90%, 95%, 98%, or 99% with SEQ ID NO: 3, and in which the polypeptide encoded by such nucleic acids has the activity and the structural characteristics of the ERK1 protein. Among such molecules of nucleic acid the one included in the sequence SEQ ID NO: 4 is to be found.

As used herein, ERK2 (also called Mitogen-activated protein kinase 1 or MAPK1) is defined as well by a nucleotides or polynucleotides sequence, which constitutes the encoding sequence of the protein included in SEQ ID NO: 5 (GeneBank M84489.1) and which would include diverse variants coming from:
a) nucleic acid molecules that encode a polypeptide which includes the amino acid sequence of SEQ ID NO: 5,
b) nucleic acid molecules whose complementary chain hybridizes with the polynucleotide sequence of a)
c) nucleic acid molecules whose sequence differs from a) and/or b) due to the degeneration of the genetic code,
d) nucleic acid molecules that encode a polypeptide which includes the amino acid sequence with an identity of at least 80%, 90%, 95%, 98%, or 99% with SEQ ID NO: 5, and in which the polypeptide encoded by such nucleic acids has the activity and the structural characteristics of the ERK2 protein. Among such molecules of nucleic acid the one included in the sequence SEQ ID NO: 6 is to be found.

As used herein, MEK is presented as a type of proteins which, among others, include MEK1 and MEK2. MEK1 (*Mitogen-Activated Protein kinase kinase 1* or MAPKK1 or MAP2K1) is encoded in a gene located in chromosome 15 (locus 15q22.1q22.3). In the context of the present invention, MEK1 is also defined by a nucleotides or polynucleotides sequence, which constitute the encoding sequence of the protein included in SEQ ID NO: 7 (GeneBank L11284.1), and which would include diverse variants coming from:
a) nucleic acid molecules that encode a polypeptide which includes the amino acid sequence of SEQ ID NO: 7,
b) nucleic acid molecules whose complementary chain hybridizes with the polynucleotide sequence of a),
c) nucleic acid molecules whose sequence differs from a) and/or b) due to the degeneration of the genetic code,
d) nucleic acid molecules that encode a polypeptide which includes the amino acid sequence with an identity of at least 80%, 90%, 95%, 98%, or 99% with SEQ ID NO: 7, and in which the polypeptide encoded by such nucleic acids has the activity and the structural characteristics of the MEK1 protein. Among such molecules of nucleic acid the one included in SEQ ID NO: 8 is to be found.

As used herein, MEK2 (*Mitogen-Activated Protein kinase kinase* 2 or MAPKK2 or MAP2K2) is encoded in a gene located in chromosome 19 (locus 19p13.3) which is defined as well by a nucleotides or polynucleotides sequence, which constitutes the encoding sequence of the protein included in SEQ ID NO: 9 (GeneBank L11285.1) and which would include diverse variants coming from:
a) nucleic acid molecules that encode a polypeptide which includes the amino acid sequence of SEQ ID NO: 9,
b) nucleic acid molecules whose complementary chain hybridizes with the polynucleotide sequence of a),
c) nucleic acid molecules whose sequence differs from a) and/or b) due to the degeneration of the genetic code,
d) nucleic acid molecules that encode a polypeptide which includes the amino acid sequence with an identity of at least 80%, 90%, 95%, 98%, or 99% with SEQ ID NO: 9, and in which the polypeptide encoded by such nucleic acids has the activity and the structural characteristics of MEK2 protein. Among such molecules of nucleic acid the one included in sequence SEQ ID NO: 10 is to be found.

As used herein, AKT is presented as a group of proteins, which participate in the control mechanisms of apoptosis (programmed cell death) and cell proliferation. Among them, the AKT1 protein (also called protein Kinase B) for example, stands out. In the context of the present invention, AKT1 is defined as well by a sequence of nucleotides or polynucleotides, that constitutes the encoding sequence of the protein included in SEQ ID NO: 11 (GeneBank M63167.1), and which would include diverse variants coming from:
a) nucleic acid molecules that encode a polypeptide which includes the amino acid sequence of SEQ ID NO: 11,
b) nucleic acid molecules whose complementary chain hybridizes with the polynucleotide sequence of a),
c) nucleic acid molecules whose sequence differs from a) and/or b) due to the degeneration of the genetic code,
d) nucleic acid molecules that encode a polypeptide which includes the amino acid sequence with an identity of at least 80%, 90%, 95%, 98%, or 99% with SEQ ID NO: 11, and in which the polypeptide encoded by such nucleic acids has the activity and the structural characteristics of the AKT1 protein. Among such molecules of nucleic acid the one included in the sequence SEQ ID NO: 12 is to be found.

As used herein, AKT2 is defined as well by a nucleotides or polynucleotides sequence, which constitute the encoding sequence of the protein included in SEQ ID NO: 13 (GeneBank M77198.1), and which would include diverse variants coming from:
a) nucleic acid molecules that encode a polypeptide which includes the amino acid sequence of SEQ ID NO: 13,
b) nucleic acid molecules whose complementary chain hybridizes with the polynucleotide sequence of a),
c) nucleic acid molecules whose sequence differs from a) and/or b) due to the degeneration of the genetic code,
d) nucleic acid molecules that encode a polypeptide which includes the amino acid sequence with an identity of at least 80%, 90%, 95%, 98%, or 99% with SEQ ID NO: 13, and in which the polypeptide encoded by such nucleic acids has the activity and the structural characteristics of the AKT2 protein. Among such molecules of nucleic acid the one included in SEQ ID NO: 14 is to be found.

As used herein, "reference sample" or "reference value" is presented as the sample used to determine the variation in the levels of expression of the proteins of the present invention. In an embodiment of the invention, the reference value is obtained from the signal obtained when using a sample of tissue obtained from an individual who presents a tumor with lack of response or poor response to the treatment of cancer with NK1 receptor antagonists. Preferably, the samples are taken from the same tissue of several non-respondent individuals, and they are combined so that the reference value reflects the average value of such molecules in the population of non-respondents. "Reference value" is the expression level of a protein in the invention (receptor NK1, ERK, AKT or truncated form of NK1 receptor) in the reference sample, or the number of cells expressing a protein of the invention in a reference sample. The reference value indicating the no response for each concrete protein must be known before implementing the method of the present invention. It is preferred that the reference sample is from the same tissue and/or obtained by the same procedure (options described above) as the sample from which the individual's response will be predicted. It is preferred that the sample stems from the same stage or from the same disease severity of the cancer, as the sample from which the individual's response will be predicted.

As used herein, "prognosis" is presented as the expected evolution of a disease, and it is referred to the valuation of the probability according to which a subject has a disease, as well as to the valuation of its onset, developmental state, evolution, or its regression, and/or the prognosis of the course of the disease in the future. As the experts in the field will understand, even if such valuation is preferred to be correct, normally it may not be correct for 100% of the subjects that will be diagnosed. The term, however, requires that a statistically significant part of the subjects can be identified as having the disease, or as having predisposition to develop it. If one part is statistically significant it can be simply determined by the expert in the field using several statistic tools of evaluation which are well known, for example, determination of confidence intervals, determination of p-values, t-Student Test, Mann-Whitney Test, etc. The preferred confidence intervals are at least 50%, at least 60%, at least 70%, at least 80%, at least 90%, at least 95%. The p-values are, preferably, 0.2, 0.1, 0.05.

With "response prediction" in the context of this invention, it is meant the determination of the probability that the patient responds favourably or unfavourably to a therapy or to a given treatment, including surgical treatment. Especially the term "prediction", as it is used here, refers to an individual evaluation of any parameter that may be useful to determine the evolution of a patient. As experts in the field will understand, the prediction of the clinical response to treatment, even if it is preferred that it be correct, does not need to be correct for 100% of the subjects to be diagnosed or evaluated. The term, however, requires that a statistically significant part of the subjects can be identified as having an increased probability of having a positive response. The expert in the field can easily determine if a subject is statistically significant using several statistic tools of evaluation which are well known, for example, determination of confidence intervals, determination of p-values, t Student Test, Mann-Whitney Test, etc. The preferred confidence intervals are at least 50%>, at least 60%>, at least 70%>, at least 80%>, at least 90%>, at least 95%>. The p-values are, preferably, 0.2, 0,1, or 0.05. The prediction of the clinical response can be done using any criteria of valuation used in oncology and known by the expert in the field.

At the same time, considering the method of the present invention, we could establish other subclassifications within this main one, facilitating therefore, the election and the establishment of therapeutic regimes or adequate treatments. This discrimination, as an expert in the field understands, does not aim to be correct in 100% of the analysed samples. Nonetheless, it requires that a statistically significant amount of the analysed samples are correctly classified. The amount that is statistically significant may be established by an expert in the field using different statistic tools, for example, determining confidence intervals, determining p-value of significance, Student's test or Fisher's discriminant functions, Mann Whitney's non-parametric measures, Spearman's correlation, logistic regression, linear regression, area under ROC curve (AUC). Preferably, the confidence intervals are at least 90%, at least 95%, at least 97%, at least 98% or at least 99%. Preferably, p-value is less than 0.1, than 0.05, than 0.01, than 0.005 or than 0.0001. Preferably, the present invention permits to correctly detect the disease in a differential form in at least 60%, more preferably in at least 70%, much more preferably in at least 80%, or even much more preferably in at least 90% of the subjects of a given group or analysed population.

In the context of the present invention the term "positive response" refers to a response of patients having cancer, which is more effective to the treatment with NK1 receptor antagonists than other patients having cancer but they do not respond in such an effective way to the treatment with NK1 receptor antagonists. The response may be referred to the characteristics of the subject's tumors. In an alternative but not mutually excluding way, the response may be referred to the clinical result of the subject. Therefore, by means of the first method of invention, it can be predicted if an individual patient will show (i) a response (R) to the treatment with NK1 receptor antagonists or (ii) will not show response (NR) to the treatment with NK1 receptor antagonists.

Always when the response is referred to the characteristics of the subject's tumors, the distinction between R and NR can be implemented on the basis of the change in the size of the lesion. For example, it is possible to define "Response" as a reduction in the total bulk of the tumor (defined as the sum of the areas of the tumor of lesions bidimensionally measured), considering as reference the values corresponding to the initial value, without the appearance of new lesions. Particularly, the response can be ≥ 5% of reduction in the total tumor bulk, such as ≥ 10% of reduction in the total tumor bulk, ≥ 15% of reduction of the total tumor bulk, ≥ 20% of reduction in the total tumor bulk , ≥ 25% of reduction in the total tumor bulk, ≥ 30% of reduction in the total tumor bulk, ≥ 35% of reduction in the total tumor bulk, ≥ 40% of reduction in the total tumor bulk , ≥ 45% of reduction in the total tumor bulk, or ≥ 50% of reduction in the total tumor bulk, defined as the sum of the areas of the tumor of the lesions bidimensionally measured. In a preferred embodiment of the present invention, the response is defined as ≥ 30% of reduction in the total tumor bulk (defined as the sum of the areas of the tumor of the lesions bidimensionally measured), considering as reference the values corresponding to the initial values, without the appearance of new lesions. The absence of response (NR) is defined as opposed to (R). Preferably, without response (NR) is defined as < 30% of reduction in the area of the tumor of one or more measurable lesions, or the appearance of at least a new lesion. This normally includes an increase, such as > 20% of increase in the area of one or more measurable lesions or the appearance of at least a new lesion.

Every time that "Response" refers to the clinical result of the subject, the "Response" can be expressed as the global survival or the progression-free survival. The survival of cancer patients is accurately expressed in general by means of the Kaplan-Meier curves, which have received their name from Edward L. Kaplan and Paul Meier, who were the ones who described them in first place (Kaplan, Meier: Amer. Statist. Assn. 53:457- 35 481).

In the present invention, it is also shown that tumors that present a mutation in the K-ras gene, present a lesser response (less effective) to the treatment with NK1 receptor antagonists.

Therefore, in other preferred implementation of this aspect of the invention, in the first method it is also determined the presence or not of mutations in the *K-ras* gene in the tumor cells of the sample in step (a), being the result an indication of a positive response (most effective response to the treatment with NK1 receptor antagonists) if the cancer cells do not present mutation in the *K-ras* gene (not mutated *K-ras* or *"wild type*").

In this invention "ras" is defined as a type of proteins that act in multiple signaling molecular pathways that regulate biological process of great importance in cancer, for instance, cell proliferation, migration, etc. One of these routes is the MAP Kinases (*Mitogen-Activated Protein Kinases*). The family of the ras proteins includes H-ras, N-ras and K-ras. K-ras protein is encoded by the *K-ras* gene, located in chromosome 12.

In the present invention it is also shown that the NK1 receptor antagonists can inhibit the growth and tumor proliferation, more effectively, in those types of tumors in which the signaling pathways of the MAP Kinases and PI3 Kinases has a normal functioning in the cells that constitute them. That is to say, they are active and the molecules constituting them do not suffer alteration, and therefore the treatment with such antagonists inhibits the proliferation of the tumor cells through the MAP Kinases and PI3 Kinases pathways, preferably by means of the inhibition of the expression of different downstream effectors in such pathways, such as for instance ERK, MEK (for the MAP Kinases pathway) and AKT (for the PI3 Kinases pathway). However, when such pathways have a non-normal functioning, the use of NK1 receptor antagonists presents a lesser effectivity.

Therefore, in other preferred implementation of this aspect of the invention, in the first method it is also determined the presence or absence of the ERK and/or MEK proteins, or the mRNA encoding thereof, in the tumor cells of the sample in step (a), being the result an indication of a positive response (most effective response to the treatment with NK1 receptor antagonists) if the cancer cells present the ERK and/or MEK proteins, or a high amount of mRNA in such proteins.

In other preferred embodiment of this aspect of the invention, in the first method it is determined the presence or absence of the AKT protein, or the mRNA encoding thereof, in the tumor cells of the sample in step (a), being the result an indication of a positive response (most effective response to the treatment with NK1 receptor antagonists) if the cancer cells present the AKT protein, or a high amount of the mRNA in such proteins.

In this invention ERK (*Extracellular-signal-regulated kinases*) is defined as a type of protein that is part of the molecular pathway of the MAP Kinases, and which participates in multiple, important cellular functions, of relevance in the physiopathology of cancer. For example ERK1 (also called Mitogen-activated protein kinase 3 or MAPK3) is a protein encoded in a gene located in chromosome 16. ERK2 (also called Mitogen-activated protein kinase 1 or MAPK1) is a protein encoded in a gene located in chromosome 22.

In this invention, MEK is defined as a type of protein that includes, among other, MEK1 and MEK2. MEK1 also called *Mitogen-Activated Protein Kinase Kinase 1,* or MAPKK1, or MAP2K1, is encoded in a gene located in chromosome 15 (locus 15q22.1-q22.3). MEK2 also called *Mitogen-Activated Protein Kinase Kinase* 2, or MAPKK2, or MAP2K2, is encoded in a gene located in chromosome 19 (locus 19p13.3).

In this invention, AKT is defined as a group of proteins that participate in the control mechanisms of apoptosis (programmed death) and of cell proliferation. Among them, two prominent examples are AKT1 (also called protein Kinase B), being encoded in a gene located in chromosome 14, and AKT2 which is encoded in a gene located in chromosome 19.

Also, in this invention is shown that tumors presenting a higher amount of truncated form of NK1 receptors have a better (more effective) response to treatment with NK1 receptors antagonists.

Therefore, in another preferred embodiment of this aspect of the invention, in the first method it is also determined the presence or absence of the truncated form of the NK1 receptor in the tumor cells of the sample in step (a), the truncated form of the NK1 receptor, or of the mRNA encoding it.

Finally, in this invention it is shown that tumor cells with alterations in the NK1 gene that determine the structural alterations that make this gene constitutively active, that is, that the receptor transmits downstream signals independently of it being coupled or not to any of its agonists, have a better response (more effective) to the treatment with NK1 receptor antagonists.

Therefore, in other preferred embodiment of this aspect of the invention, in the first method it is also determined if the tumor cells of the step (a) present a constitutively activated form of the NK1 receptor, being the result an indication of a positive response (most effective response to the treatment with NK1 receptor antagonists) if tumor cells present a constitutively activated form of the NK1 receptor. More preferably, the determination of the presence or absence of a constitutively activated form of the NK1 receptor in the tumor cells of the sample in step (a) , it is performed by exposing the cells to the substance P, considering that the cells present a constitutively activated form of the NK1 receptor, if they present a low proliferative response when exposed to the substance P.

The expression levels of genes will reveal a specific gene expression profile. The term "expression level", also called "gene product quantity" refers to the biochemical material, either RNA or protein, that results from the expression of a gene. Sometimes it is used a measure of the quantity of gene product to infer how active a gene is. With "gene profile expression" it is referred to the genetic profile obtained after the quantification of the mRNA and/or of the protein produced by the genes of interest or biomarkers, that is, by genes used as biological markers in the present invention, in an isolated sample. The gene expression profile is executed, preferably, by determining the mRNA level stemming from its transcription, having previously extracted the total RNA present in the isolated sample, which can be done by using protocols known in the state of the art. The determination of the mRNA level derived from the transcription of the genes used as biological markers in the present invention can be executed, for example, without limiting, by means of the amplification by the polymerase chain reaction (PCR), reverse transcription in combination with the polymerase chain reaction (RT-PCR), quantitative RT-PCR (RTqPCR), reverse transcription in combination of ligase chain reaction (RT-LCR), or any other method of amplification of nucleic acids; serial analysis of gene expression (SAGE, SuperSAGE); DNA chips produced with oligonucleotides deposited by any mechanisms; DNA microarrays produced with oligonucleotides synthetized *in situ* by means of photolithography or by any other mechanism; in situ hybridization using specific probes marked by any marking method; by means of electrophoresis gels; by means of membrane transference and hybridization with a specific probe; by means of nuclear magnetic resonance or any other diagnostic imaging technique using paramagnetic nanoparticles or any other type of detectable antibody-functionalized nanoparticles or by any other means. The gene expression profile could also be obtained by means of the detection and/or quantification of the proteins resulting from the translation of the mRNA derived from the transcription of the genes used as biological markers in the present invention, by means for example, and without limiting, by immunodetection by western blot. The quantitative detection of the expression of the genes used as biological markers in the present invention can be executed more preferably by means of real time PCR (RT-PCR or RTqPCR). The detection in real time of the amplified products can be carried on by means of the use of fluorescent molecules that are interspersed in the double stranded DNA or by means of the hybridization with different types of probes.

Therefore, the detection of the proteins levels or gene expression level can be done by any of the known techniques by the expert in the field. Hence, in other preferably embodiment of this aspect in the invention,
a) the detection of the levels of expression of NK1 receptor, or the mRNA encoding the NK1 receptor, or
b) the presence or absence of mutations in the *K-ras* gene,
c) the presence of the ERK protein, and/or
d) the presence of the MEK protein, and/or
e) he presence of the AKT, or
f) the presence or absence of the truncated form of the NK1 receptor,
is implemented by means of
i. genetic profiling procedure, such as a micro-matrix, and/or
ii. procedure including PCR, such as a real time PCR;
   and/or
iii. Northern blot transference, and/or
iv. immunohistochemical procedure.

In other preferred embodiment, the sample is a recent tissue, a paraffin embedded tissue, or RNA extracted from the tissue of a patient with cancer. More preferably, the first method of the invention is implemented *in vitro* using a sample originated in the human subject, in which at the moment of taking the sample from, said subject has been treated with an NK1 receptor agonist.

In other preferred embodiment, the detection of the levels of gene expression is executed by means of RTqPCR.

In other preferred embodiment, the detection of NK1, ERK, MEK, AKT proteins levels and/or the truncated NK1 receptor, is executed by means of immunological techniques. In a more preferred implementation, the immunological techniques are based on precipitating reactions, based on agglutinating reactions, immunolabelling, radioimmunoassay and radioimunometrics techniques, ELISA (*Enzyme-Linked Immunosorbent Assay*), or in any of their combinations. In other, more preferred embodiment, the immunological techniques include immunomarking. In other, even more preferred embodiment, the immunolabelling technique is selected among immunolabelling with antibodies coupled to enzymes, immunolabelling with antibodies coupled to fluorochromes, or cytometry. Even more preferably, the cytometry is flow cytometry.

In other preferred embodiment of this aspect of the invention, the human subject suffers from a type of cancer selected among the following: gastric carcinoma, preferably gastric adenocarcinoma, colon carcinoma, preferably colon adenocarcinoma, pancreas carcinoma, preferably pancreatic adenocarcinoma, renal cell carcinoma, preferably clear cell renal cell carcinoma, breast carcinoma, preferably breast adenocarcinoma, ovarian carcinoma, preferably ovarian adenocarcinoma, endometrial carcinoma, carcinoma of the uterine cervix, lung carcinoma, preferably non-small-cell lung cancer and/or small-cell lung cancer, thyroid carcinoma, preferably papillary thyroid carcinoma and/or follicular thyroid carcinoma, bladder carcinoma, preferably transitional cell carcinoma of the urinary bladder, prostate carcinoma, cancer of glial origin of the central nervous system (glioma), sarcomas, preferably fibrosarcoma, malignant fibrous histiocytoma and Edwing sarcoma, melanoma, embryonal cancers, preferably neuroblastoma and hematological cancers, preferably B-cell or T-cell leukemia, non-Hodgkin lymphoma, preferably non Hodgkin lymphoma B-cell or T-cell types, Burkitt lymphoma, Hodgkin lymphoma and multiple myeloma.

In other preferred embodiment of this aspect of the invention, the antagonist of the NK1 receptor are non-peptidic antagonists, and even more preferably, the non-peptidic antagonists are selected among: Aprepitant, Vestipitant, Casopitant, Vofopitant, Ezlopitant, Lanepitant, LY-686017, L-733,060, L-732,138, L-703,606, WIN 62,577, TAK-637, y R673, CP-100263, WIN 51708, CP-96345, L-760735, CP-122721, L-758298, L-741671, L-742694, CP-99994, T-2328, or any of their combinations.

In other preferred embodiment of this aspect of the invention, the antagonists of the NK1 receptor are peptidic antagonists, and even more preferably, they are selected among antibodies or fragments of them directed against the NK1 receptor.

As used herein, "non-peptidic antagonist of the NK1 receptor" refers to any non-peptidic substance having a sufficient size and the adequate configuration to couple to NK1 receptor, so that they can inhibit its normal functioning, avoiding as well that the SP or other agonists of these receptors can couple to the mentioned receptors. Preferably, in the present invention we have tested the following non-peptidic antagonists of commercialized NK1 receptors: L-733,060 ((2S,3S)-3-[(3,5-bis(Trifluoromethyl)phenyl)methoxy]-2-phenylpiperidine hydrochloride) (Sigma-Aldrich), L-732,138 (N-Acetyl-L-tryptophan 3,5-bis(trifluoromethyl)benzyl ester) (Sigma-Aldrich), L-703,606 (cis-2-(Diphenylmethyl)-N-[(2-iodophenyl)methyl]-1-azabicyclo[2.2.2]octan-3-amine oxalate salt) (Sigma-Aldrich), WIN 62,577 (Sigma-Aldrich), Aprepitant or MK 869 or L-754030 (MSD), TAK-637 (Takeda/Abbot), Vestipitant or GW597599 (GSK), Casopitant or GW679769 (GSK) and R673 (Roche). CP-100263, WIN 51708, CP-96345, L-760735. Similarly, other compositions of non-peptide NK1 receptor antagonists. and of SP can be used, such as: Vofopitant or GR-205171 (Pfizer), Ezlopitant or CJ -11974 (Pfizer), CP-122721 (Pfizer), L-758298 (MSD), L-741671, L-742694, CP-99994, Lanepitant or LY-303870, T-2328, LY-686017. The preferred ones are: Aprepitant or MK 869 or L-754030 (MSD), Vestipitant or GW597599 (GSK), and Casopitant or GW679769 (GSK).

Antibodies are peptide compositions with the ability of coupling in a highly selective way to diverse organic molecules. Their coupling to some cellular receptors induces a modification in their activity, which can determine a modification in the regular physiological activities of the cellular metabolism.

As it is used here "antibody against NK1 receptor" refers to any polyclonal or monoclonal antibody or fragments thereof against NK1 receptors. A fragment of antibody is a part of an antibody against NK1 receptor having a sufficient size and the adequate configuration to couple to an epitope that is present in NK1 receptors and thus modify its normal functioning, avoiding as well that the SP or other agonists of these receptors can couple to the mentioned receptors.

The use of monoclonal antibodies directed against the extracellular domain of diverse cellular receptors, as treatment against cancer, is widely spread in the clinical field. Among these therapeutic monoclonal antibodies approved for use in oncology, some of them are to be highlighted: trastuzumab, which is an anti-ErbB2/HER2 for breast cancer, cetuximab, which is an anti-ErbB1/EGFR for colon cancer, and bevacizumab, which is an anti-VEGFR (receptor of the vascular endothelial growth factor) for diverse types of cancer (Adams GP. et al., 2005).

With "cancer" it is referred to a malign tumor of potential, unlimited growth which locally expands by means of invasion, and systemically by means of metastasis. According to the present invention, the non-peptidic antagonist of the NK1 receptor will be given to individuals having cancer.

In the context of the present invention, the use of "drugs seeking to modify the functioning of NK1 receptors" is referred to non-peptidic antagonist molecules or antibodies, or fragments thereof, which act on NK1 receptors and modify their functioning, seeking to provoke an inhibition of the proliferation producing death and/or apoptosis in tumor cells in mammals, including humans, either by acting directly on the physiological mechanisms of tumor cells or by modifying the tumor microenvironment.

In the context of the present invention, the disease is cancer, preferably gastric carcinoma, preferably gastric adenocarcinoma, colon carcinoma, preferably colon adenocarcinoma, pancreas carcinoma, preferably pancreatic adenocarcinoma, renal cell carcinoma, preferably clear cell renal cell carcinoma, breast carcinoma, preferably breast adenocarcinoma, ovarian carcinoma, preferably ovarian adenocarcinoma and/or ovarian carcinoma, endometrial carcinoma, carcinoma of the uterine cervix, lung carcinoma, preferably lung adenocarcinoma, non-small-cell lung cancer and/or small-cell lung cancer, thyroid carcinoma, preferably metastasizing papillary thyroid carcinoma and/or follicular thyroid carcinoma, bladder carcinoma, preferably urine bladder carcinoma and/or transitional cell carcinoma of the urinary bladder, prostate carcinoma, cancer of glial lineage of the central nervous system (glioma), sarcomas, preferably fibrosarcoma, malignant fibrous histiocytoma and Edwing sarcoma (although it is also useful in human endometrial stromal sarcoma, osteosarcoma and/or rhabdomyosarcoma), melanoma, embryonal cancers, preferably neuroblastoma (although it is also useful in medulloblastoma, retinoblastoma, nephroblastoma and/or hepatoblastoma), and hematological cancers, preferably B-cell or T-cell leukemia, non Hodgkin lymphoma, preferably non Hodgkin lymphoma B-cell or T-cell types, Burkitt lymphoma, Hodgkin lymphoma, leukemias, preferably B-cell or T-cell types, and multiple myeloma.

### Use of pharmaceutical compositions comprising an antagonist of the NK1 receptor

Another aspect of the present invention refers to the use of the expression level of the NK1 receptor in a sample of tumor cells as an indicator in an *in vitro* method to predict if a human suffering from cancer will have a positive response to treatment with a pharmaceutical composition comprising an antagonist of the NK1 receptor.

In a preferred embodiment of this aspect of the invention, the antagonist of the NK1 receptor are non-peptidic antagonists. More preferably, the non-peptidic antagonists are selected among: Aprepitant, Vestipitant, Casopitant, Vofopitant, Ezlopitant, Lanepitant, LY-686017, L-733,060, L-732,138, L-703,606, WIN 62,577, TAK-637, y R673, CP-100263, WIN 51708, CP-96345, L-760735 , CP-122721, L-758298, L-741671, L-742694, CP-99994, T-2328, or combinations thereof.

Non-peptidic antagonists of the NK1 receptor are, but not being limited to: L-733,060 ((2S,3S)-3-[(3,5-bis(Trifluoromethyl)phenyl)methoxy]-2-phenylpiperidine hydrochloride) (Sigma-Aldrich), L-732,138 (N-Acetyl-L-tryptophan 3,5-bis(trifluoromethyl)benzyl ester) (Sigma-Aldrich), L-703,606 (cis-2-(Diphenylmethyl)-N-[(2-iodophenyl)methyl]-1-azabicyclo[2.2.2]octan-3-amine oxalate salt) (Sigma-Aldrich), WIN 62,577 (Sigma-Aldrich), Aprepitant or MK 869 or L-754030 (MSD), TAK-637 (Takeda/Abbot), Vestipitant or GW597599 (GSK), Casopitant or GW679769 (GSK) and R673 (Roche). CP-100263, WIN 51708, CP-96345, L-760735. Similarly, other compositions of non-peptidic antagonists of the NK1 receptor and of SP can be used, such as: Vofopitant or GR-205171 (Pfizer), Ezlopitant or CJ -11974 (Pfizer), CP-122721 (Pfizer), L-758298 (MSD), L-741671, L-742694, CP-99994, Lanepitant or LY-303870, T-2328, LY-686017. The preferred compositions are: Aprepitant or MK 869 or L-754030 (MSD), Vestipitant or GW597599 (GSK), and Casopitant or GW679769 (GSK), or combinations thereof.

In another preferred embodiment of this aspect of the invention, the antagonists of the NK1 receptor are peptidic antagonists. More preferably, the peptidic antagonists are selected among antibodies or fragments of them directed against the NK1 receptor.

These antagonists of the NK1 receptor, both peptidic and non-peptidic, can be used alone, combined among themselves, or with diverse anticancerigen agents that are selected, but without being limited to, among any of the following: Chlorambucil, Melphalan, Aldesleukin, 6-mercaptopurine, 5-fluoruracil, Ara-c, Bexarotene, Bleomicin, Capecitabine, Carboplatin, Cisplatin, Docetaxel, Doxorubicine, Epirubicine, Fludarabine, Irinotecan, Methotrexate, Mitoxantrone, Oxaliplatin, Paclitaxel, Rituximab, Etoposide, Teniposide, Vincristine, Vinblastine, Vinorelbine, Imatinib, Erlotinib, Cetuximab, and Trastuzumab, or any combination thereof.

The terms "polynucleotides" and "nucleic acid" are used here interchangeably, both being referred to polymeric forms of nucleotides of any length, both ribonucleotides (RNA) and deoxyribonucleotide (DNA). The terms "amino acid sequence", "peptide", "oligopeptide", "polypeptide" and "protein" are used here interchangeably, and they refer to a polymeric sequence of amino acids of any length, which can by encoding or not encoding, chemically or biochemically modified.

Unless otherwise defined, all technical and scientific terms used here have the same meaning as usually understood by an expert in the field of the invention. Methods and similar materials or equivalent to those described here can be used in the application of the present invention. Along the description and claims the word "comprises" and its variants do not have a limitative nature, and therefore they do not aim to exclude other technical characteristics, additives, components or steps. On the contrary, the word "consist" and its variants, do present a limitative nature, being exclusively referred to technical characteristics, additives, components or steps accompanying it. To the experts in the field, other objects, advantages, and characteristics of the invention will partly come from the description and partly from the application of the invention. The following examples and drawings are given as illustrations, and they do not aim to be limitative of the present invention.

### Examples of the invention

In what follows, examples are shown as an illustration, without being limitative to the present invention, which reflects the advantages of the invention. Examples falling outside the scope of the claims are provided for reference purposes.

### Example 1. Cancer cells with the higher expression of NK1 receptor present a more effective response to the treatment with antagonists of the NK1 receptor.

To check that cancer cells with the higher expression of NK1 receptor (higher percentage of cells with expression of such receptor or higher intensity in its expression) showed a more effective response to the treatment with antagonists of the NK1 receptor, it was taken primary tumor cells stemming from patients' tumors, and the presence of NK1 receptor in them was determined by means of western-blot and immunohistochemical techniques, and they were cultured in presence of diverse antagonists of the NK1 receptor. The different tumors and the patients' characteristics are shown in Table 1.

**Tabla 1. Characteristics of the patients and of the tumor samples obtained for cell culture.**

| **Type of Tumor** | **Number of cases** | **Sex (Man /Woman)** | **Age** | **Histological Type** |
|---|---|---|---|---|
| Gastric carcinoma | 10 | 4/6 | 52±3 | Adenocarcinoma |
| Colon carcinoma | 12 | 5/7 | 58±4 | Adenocarcinoma |
| Pancreatic carcinoma | 9 | 4/5 | 52±7 | Adenocarcinoma |
| Renal cell carcinoma | 9 | 5/4 | 52±7 | Clear Cell Carcinoma |
| Breast carcinoma | 9 | 0/9 | 49±6 | Ductal Adenocarcinoma |
| Ovarian carcinoma | 9 | 0/9 | 52±5 | Adenocarcinoma |
| Endometrium carcinoma | 9 | 0/9 | 61±5 | Adenocarcinoma |
| Carcinoma of the uterine cervix | 9 | 0/9 | 35±4 | Squamous Cell Carcinoma |
| Lung carcinoma | 10 | 8/2 | 59±5 | Small Cell Carcinoma |
| Lung Lung carcinoma | 10 | 8/2 | 66±4 | Non Small Cell Carcinoma |
| Thyroid carcinoma | 9 | 2/7 | 46±5 | Follicular Carcinoma |
| Thyroid carcinoma | 9 | 2/7 | 38±4 | Papillary Carcinoma |
| Urinary bladder | 9 | 7/2 | | Urotelial (transicional) Cell Carcinoma |
| Prostate carcinoma | 9 | 9/0 | 70±5 | Adenocarcinoma (Gleasson 3+4) |
| Glioma | 9 | 6/3 | 59±4 | Astrocytoma |
| Fibrosarcoma | 9 | 6/3 | 60±4 | Fibrosarcoma (histologic grade 2) |
| Malignant Fibrous Histiocytoma. | 9 | 6/3 | 68±5 | Malignant Fibrous Histiocytoma (histologic grade 3). |
| Ewing Sarcoma | 9 | 5/4 | 33±4 | Ewing Sarcoma |
| Melanoma | 10 | 5/5 | 56±4 | Nodular Melanoma. |
| Neuroblastoma | 9 | 5/4 | 10±5 | Neuroblastoma |
| Leukemia B | 9 | 6/3 | 24±4 | Cronic Leukemia B |
| Leukemia T | 9 | 6/3 | 31±5 | Cronic LeuKemia T |
| Non Hodgkin Lymphoma B | 9 | 5/4 | 51±4 | Diffusse B Lymhoma |
| Non Hodgkin Lymphoma T | 9 | 5/4 | 35±5 | Diffusse T Lymhoma |
| Burkitt Lymphoma | 9 | 5/4 | 25±4 | Burkitt Lymhoma |
| Hodgkin lymphoma | 9 | 5/4 | | Hodgkin lymphoma |
| Myeloma | 9 | 5/4 | 61±3 | Multiple Myeloma |

**Cell cultures.** To obtain tumor cells from human primary tumors we used the method described in De Bari et al. (De Bari et al. 2001). Briefly, small pieces of the tumors were digested into a hyaluronidase solution (Sigma-Aldrich, USA) at a concentration of 1 mg/ml during 15 minutes at 37°C, and then they were treated with 6 mg/ml of collagenase type IV (Invitrogen) during 2 hours at 37°C. Consecutively, cells were washed, and resuspended in the DMEM culture medium with high concentration of glucose (Dulbecco's Modified Eagle's Medium, Invitrogen) supplemented with 1% of antibiotics-antimycotics (Invitrogen) and with 1% of sodium pyruvate (Invitrogen), and the cells were seeded in culture slides at a concentration of 10.000 cells per square centimeter. When cells reached confluence, the adherent cells were separated using sterile trypsin at 0,5% (Invitrogen) and they were used among passages 3 and 9. For the assays shown next, tumor cells were seeded on slides at a concentration of 25.000 cells per well. A total of 6 wells containing the tumor cells were used as survival control. Other groups of 6 wells containing the tumor cells were cultivated in the presence of the different antagonists of the NK1 receptor, such as Aprepitant, Vestipitant, Casopitant, and antibodies against such receptor (obtained from Sigma-Aldrich, catalogue number S8305). All the cultures were kept during 48 hours and later on recollected for their analysis.

**Western-Blot.** It was checked in all tumor cell lines the presence of the NK1 receptor by means of Western-Blot. Briefly, the extraction of total proteins was made from samples obtained from cellular cultures. The cells were lysed using methods easily known in the state of the art, and their concentration was quantified by means of a commercial kit "Protein Assay" from (Bio-Rad Laboratories, S.A. Madrid), following the instructions provided. From each sample, 50 mg of proteins were separated by 10% SDS-polyacrylamide electrophoresis and were electrophoretically transferred to polyvinylidene fluoride (PVDF) membranes. Such membranes were incubated overnight in a blocking buffer solution (5% skimmed milk in phosphate buffer -PBS- with 0.1 of de Tween-PBST), followed by an incubation overnight with the primary antibodies diluted 1/4000 in PBST buffer. As primary antibody, the anti-NK1 antibody (S8305, Sigma-Aldrich) was used, which recognizes amino acids 393-407 of the carboxy terminal domain of NK1 receptor. Then, the membranes were washed with phosphate saline buffer (PBS) and phosphate saline buffer in the presence of detergent Tween-20 (PBST), and the membranes were incubated with a secondary antibody coupled with horseradish peroxidase during 2 hours at room temperature (dilution 1:10000). To confirm that the same amount of protein had been charged, membranes were incubated with the monoclonal antibody anti-β-tubulin antibody. The detection of the antibodies was performed with a chemiluminescence reaction (ECL Western Blot detection; Amersham Life Science, United Kingdom).

**Immunohistochemistry techniques.** The presence of NK1 receptors was also analyzed using immunohistochemistry techniques. Briefly, a sample of each of the cultures of the cell lines used in the present invention, was centrifuged (5 minutes at 1.500 rpm) and the pellet obtained was dehydrated using increasing concentrations of ethanol and, finally, xylol. Then, said dehydrated samples were embedded in paraffin, creating a block of cells. Such paraffin blocks were cut with a microtome at 5 microns thickness, and were placed on microscope slides adequate to perform immunohistochemistry techniques. Consecutively, they were de-paraffinized by immersing them in xylol, to be finally hydrated using a solution containing decreasing concentrations of ethanol, and finally submerged them into water. Then, these samples were underwent a treatment inside a pressure cooker in citrate buffer 10x pH 6.0, to obtain a higher exposition of the antigens. After this, the samples were left to cool down at room temperature for 10 minutes. The endogenous peroxidase activity was blocked with 3% peroxide of hydrogen for 30 minutes at room temperature. After washing the samples with 0.05 M of Tris buffer, they were incubated with 10% non-immune pork serum at for 30 minutes at room temperature. To analyze the expression of NK1 receptors, the cellular samples were incubated in the presence of anti-NK1 antibodies (S8305, Sigma-Aldrich) diluted 1:1000, overnight at 4°C. Afterwards, the samples were washed in Tris buffer 0.05 M at room temperature. The next step was the addition of Envision System-HRP (Dako) reagents for 30 minutes at room temperature. Then, the samples were washed again in Tris buffer 0.05 M and the immunoreactivity was visualized through light microscopy with a chromogenic solution containing 3,3'-Diaminobenzidine (DAB+; Dako, USA). To differentiate the cell nuclei, they were slightly stained with hematoxylin. As negative control, it was used samples not incubated with the primary antibody anti-NK1, which was replaced by non-immune serum. All the samples were evaluated.

To value the immunostaining in each of the cases six cuts were performed to be analyzed by the immunohistochemistry technique. In each of the six cuts, it was counted cells in 20 fields (magnification 400x) using an Olympus microscope (model CX31). In each field it was counted the total number of cells and the number of cells presenting immunostaining, calculating afterwards the percentage of cells which presented immunostaining. The tumors were distributed in groups according to the percentage of cells that presented immunostaining for the NK1 receptor: group 1 wherein the immunostaining is observed in less than 20% of cancer cells, group 2 wherein the immunostaining is observed between 20 and 80% of the cancer cells, and group 3 wherein the immunostaining is observed in more than 80% of the cancer cells. Additionally, the intensity of the immunostaining was also evaluated, grading it from 1 to 3 (in comparison to the control). As control it was used a sample of healthy tissue (controls used in each case are detailed in Table 3). The level of intensity 1 was assigned to those cases wherein more than 80% of tumor cells presented immunostaining level similar to the control group, and 20% of them, higher intensity level than the control. The level of intensity 2 was assigned to those cases wherein between 40 and 80% of tumor cells presented immunostaining intensity level higher than the control. The level of intensity 3 was assigned to those cases wherein more than 80% of the tumor cells presented an immunostaining intensity level higher than the control.

In Table 2 are displayed the types of tumors from which the samples for the cell cultures were extracted, and the distribution of cases according to the intensity of the immunostaining, determined using immunohistochemistry techniques.

**Table 2. Characteristics of the types of cancers for which the samples for the cell cultures were obtained, and distribution of cases according to the percentage of cancer cells that presented immunostaining for the NK1 receptor, using immunohistochemistry techniques.**

| **Histologic Tumor Type** | **Number of cases.** | **Number of cases in group 1 (expression in less than 20% of the cells).** | **Number of cases in group 2 (expression in 20-80% of the cells).** | **Number of cases in group 3 (expresión in more than 80% of the cells).** |
|---|---|---|---|---|
| Gastric carcinoma | 10 | 4 | 3 | 3 |
| Colon carcinoma | 12 | 4 | 4 | 5 |
| Pancreatic carcinoma | 9 | 3 | 3 | 3 |
| Renal cell carcinoma | 9 | 3 | 3 | 3 |
| Breast carcinoma | 9 | 3 | 3 | 3 |
| Ovarian carcinoma | 9 | 3 | 3 | 3 |
| Endometrium carcinoma | 9 | 3 | 3 | 3 |
| Carcinoma of the uterine cervix | 9 | 3 | 3 | 3 |
| Lung carcinoma (Non Small Cell type) | 10 | 3 | 3 | 4 |
| Lung carcinoma | 10 | 3 | 4 | 3 |
| Thyroid carcinoma (follicular type) | 9 | 3 | 3 | 3 |
| Thyroid carcinoma (papillary type) | 9 | 3 | 3 | 3 |
| Urinary bladder carcinoma | 9 | 3 | 3 | 3 |
| Prostate carcinoma | 9 | 3 | 3 | 3 |
| Glioma | 9 | 3 | 3 | 3 |
| Fibrosarcoma | 9 | 3 | 3 | 3 |
| Malignant Fibrous Histiocytoma. | 9 | 3 | 3 | 3 |
| Ewing Sarcoma | 9 | 3 | 3 | 3 |
| Melanoma | 10 | 3 | 3 | 4 |
| Neuroblastoma | 9 | 3 | 3 | 3 |
| Leukemia B | 9 | 3 | 3 | 3 |
| Leukemia T | 9 | 3 | 3 | 3 |
| Non Hodgkin Lymphoma B | 9 | 3 | 3 | 3 |
| Non Hodgkin Lymphoma T | 9 | 3 | 3 | 3 |
| Burkitt Lymphoma | 9 | 3 | 3 | 3 |
| Hodgkin lymphoma | 9 | 3 | 3 | 3 |
| Myeloma | 9 | 3 | 3 | 3 |

Table 3 shows the types of tumors from which the samples for cell cultures were extracted, the control used to value the intensity of the immunohistochemical expression of the NK1 receptor, and the distribution of cases according to the intensity of the immunostaining.

**Table 3. Characteristics of the patients from whom the samples for cell cultures were obtained, types of tumors from which the samples for cell cultures were obtained, control used to value the intensity of the immunohistochemical expression of the NK1 receptor, and distribution of the cases according to the immunostaining intensity.**

| **Histologic Type of Tumor** | **Control Cells (Normal /Nontumors Cells)** | **Number of cases (total)** | **Number of cases with intensity grade 1** | **Number of cases with intensity grade 2** | **Number of cases with intensity grade 3** |
|---|---|---|---|---|---|
| Gastric carcinoma | Cells of the normal Gastric mucosae. | 10 | 3 | 4 | 3 |
| Colon carcinoma | Cells of the normal colonic mucosae. | 12 | 4 | 5 | 3 |
| Pancreatic carcinoma | Ductal Cells of pancreas. | 9 | 3 | 3 | 3 |
| Renal cell carcinoma | Ductal Cells of kidney. | 9 | 3 | 3 | 3 |
| Breast carcinoma | Ductal Cells of the breast. | 9 | 3 | 3 | 3 |
| Ovarian carcinoma | Cells of the surface of ovary. | 9 | 3 | 3 | 3 |
| Endometrium carcinoma | Glandular cells of the endometrium | 9 | 3 | 3 | 3 |
| Carcinoma of the uterine cervix | Cervical Squamous cells of cervix | 9 | 3 | 3 | 3 |
| Lung carcinoma (non small cell type) | Cells of pulmonary bronchus | 10 | 3 | 4 | 3 |
| Lung carcinoma (small cell type) | Cells of pulmonary bronchus | 10 | 4 | 3 | 3 |
| Thyroid carcinoma (folliculary type) | Follicular cells of thyroid. | 9 | 3 | 3 | 3 |
| Thyroid carcinoma (papillary type) | Follicular cells of thyroid. | 9 | 3 | 3 | 3 |
| Urinary bladder carcinoma | Epithelial cells of urinary bladder. | 9 | 3 | 3 | 3 |
| Prostate carcinoma | Glandular cells of prostate. | 9 | 3 | 3 | 3 |
| Glioma | Astrocytes of the central nervous system | 9 | 3 | 3 | 3 |
| Fibrosarcoma | Fibroblasts of the normal soft tissues | 9 | 3 | 3 | 3 |
| Malignant Fibrous Histiocytoma. | Fibroblasts of the normal soft tissues | 9 | 3 | 3 | 3 |
| Ewing Sarcoma | Fibroblasts of the normal soft tissues | 9 | 3 | 3 | 3 |
| Melanoma | Melanocytes of skin | 10 | 3 | 4 | 3 |
| Neuroblastoma | Neurons of central nervous system | 9 | 3 | 3 | 3 |
| Leukemia B | Lymphocytes of blood. | 9 | 3 | 3 | 3 |
| Leukemia T | Lymphocytes of blood. | 9 | 3 | 3 | 3 |
| Non Hodgkin Lymphoma B | Lymphocytes of blood. | 9 | 3 | 3 | 3 |
| Non Hodgkin Lymphoma T | Lymphocytes of blood. | 9 | 3 | 3 | 3 |
| Burkitt Lymphoma | Lymphocytes of blood. | 9 | 3 | 3 | 3 |
| Hodgkin lymphoma | Lymphocytes of blood. | 9 | 3 | 3 | 3 |
| Myeloma | Plasma Cells | 9 | 3 | 3 | 3 |

It is appreciated an increase of the inhibition of cell proliferation in the cancer cell cultures that presented a higher percentage of cells with immunostaining for NK1 receptor by immunohistochemistry. In Tables 4 to 7 it can be seen how in cultures treated with Aprepitant (Table 4), Casopitant (Table 5), Vestipitant (Table 6), and antibodies against NK1 receptor - obtained from Sigma-Aldrich, catalogue number S8305- (Table 7), a higher inhibition is produced in cell proliferation of those cancer cells cultures that present a higher percentage of immunostaining of NK1 receptor. Therefore, when the percentage of cancer cells expressing NK1 receptor increases, the response to the treatment with antagonists of the NK1 receptor is higher. It is noted an increase in the inhibition of cell proliferation in cancer cells cultures that presented higher intensity of NK1 receptor immunostaining with immunohistochemistry. In Tables 8 to 11 it can be seen how in cultures treated with Aprepitant (Table 8), Casopitant (Table 9), Vestipitant (Table 10), and antibodies against the NK1 receptor-obtained from Sigma-Aldrich, catalogue number S8305- (Table 11), higher inhibition is noticed in the cell proliferation of those cancer cell cultures with a higher percentage of immunostaining of NK1 receptor. Therefore, when the intensity of NK1 receptor expression of is higher, the response to the treatment with antagonist of the NK1 receptor is higher. In the experiments reflected in Tables 4 to 11, Aprepitant, Casopitant and Vestipitant were used at 1 micromolar concentration. The antibody against NK1 receptor was used at 1/100 concentration. The percentages of inhibition are expressed in percentage of variation regarding the control ± standard deviation.

**Table 4. Percentage of inhibition of cell cultures treated with Aprepitant in relation to percentage of cells presenting immunostaining for NK1 receptor.**

| **Type of Tumor** | **Number of cases** | **Group 1 (expression in less than 20% of the cells)** | **Group 2 (expression in 20-80% of the cells)** | **Group 3 (expression in more than 80% of the cells)** |
|---|---|---|---|---|
| Gastric carcinoma | 100 | 10±3 | 45±3 | 92±3 |
| Colon carcinoma | 100 | 11±4 | 44±2 | 95±4 |
| Pancreatic carcinoma | 100 | 12±3 | 47±1 | 94±6 |
| Renal cell carcinoma | 100 | 10±3 | 45±4 | 93±6 |
| Breast carcinoma | 100 | 11±1 | 46±3 | 94±5 |
| Ovarian carcinoma | 100 | 11±2 | 45±4 | 93±3 |
| Endometrium carcinoma | 100 | 12±2 | 49±2 | 95±4 |
| Carcinoma of the uterine cervix | 100 | 10±2 | 48±5 | 95±5 |
| Lung carcinoma (non small cell type) | 100 | 9±3 | 41±3 | 94±7 |
| Lung carcinoma (small cell type) | 100 | 10±2 | 42±3 | 97±6 |
| Thyroid carcinoma (folliculary type) | 100 | 10±2 | 45±2 | 97±5 |
| Thyroid carcinoma (papillary type) | 100 | 10±3 | 47±3 | 97±6 |
| Urinary bladder carcinoma | 100 | 10±2 | 48±4 | 96±5 |
| Prostate carcinoma | 100 | 11±3 | 46±6 | 95±5 |
| Glioma | 100 | 11±4 | 47±4 | 94±6 |
| Fibrosarcoma | 100 | 10±3 | 48±3 | 96±5 |
| Malignant Fibrous Histiocytoma | 100 | 11±2 | 47±2 | 95±6 |
| Ewing Sarcoma | 100 | 11±2 | 48±6 | 94±6 |
| Melanoma | 100 | 11±3 | 49±5 | 96±4 |
| Neuroblastoma | 100 | 10±3 | 44±5 | 97±5 |
| Leukemia B | 100 | 11±2 | 43±4 | 95±4 |
| Leukemia T | 100 | 11±3 | 46±6 | 97±5 |
| Non Hodgkin Lymphoma B | 100 | 10±2 | 47±4 | 96±5 |
| Non Hodgkin Lymphoma T | 100 | 11±3 | 48±5 | 95±6 |
| Burkitt Lymphoma | 100 | 10±2 | 45±6 | 96±4 |
| Hodgkin lymphoma | 100 | 10±3 | 49±3 | 94±5 |
| Myeloma | 100 | 11±2 | 46±5 | 95±4 |

**Table 5. Percentage of inhibition of cell cultures treated with Casopitant in relation to the percentage of cells that presented immunostaining for NK1 receptor.**

| **Type of Tumor** | **Number of cases.** | **Group 1 (expression in less than 20% of the cells).** | **Group 2 (expression in 20-80% of the cells).** | **Group 3 (expression in more than 80% of the cells).** |
|---|---|---|---|---|
| Gastric carcinoma | 100 | 12±4 | 46±2 | 90±2 |
| Colon carcinoma | 100 | 13±3 | 46±3 | 92±3 |
| Pancreatic carcinoma | 100 | 10±4 | 45±2 | 92±4 |
| Renal cell carcinoma | 100 | 16±4 | 46±3 | 91±5 |
| Breast carcinoma | 100 | 15±3 | 47±4 | 92±3 |
| Ovarian carcinoma | 100 | 14±3 | 49±3 | 94±4 |
| Endometrium carcinoma | 100 | 16±3 | 46±3 | 92±3 |
| Carcinoma of the uterine cervix | 100 | 16±3 | 47±4 | 93±3 |
| Lung carcinoma (non small cell type) | 100 | 10±2 | 46±2 | 92±5 |
| Lung carcinoma (small cell type) | 100 | 12±2 | 45±2 | 93±4 |
| Thyroid carcinoma (folliculary type) | 100 | 14±3 | 44±3 | 94±4 |
| Thyroid carcinoma (papillary type) | 100 | 16±2 | 44±2 | 95±5 |
| Urinary bladder carcinoma | 100 | 17±3 | 45±3 | 94±6 |
| Prostate carcinoma | 100 | 16±2 | 45±4 | 93±4 |
| Glioma | 100 | 16±3 | 46±4 | 93±5 |
| Fibrosarcoma | 100 | 15±2 | 47±5 | 93±4 |
| Malignant Fibrous Histiocytoma. | 100 | 14±3 | 46±4 | 94±5 |
| Ewing Sarcoma | 100 | 15±2 | 47±5 | 93±5 |
| Melanoma | 100 | 15±3 | 46±4 | 93±5 |
| Neuroblastoma | 100 | 16±2 | 45±4 | 94±4 |
| Leukemia B | 100 | 17±3 | 46±5 | 94±6 |
| Leukemia T | 100 | 16±2 | 47±5 | 95±3 |
| Non Hodgkin Lymphoma B | 100 | 15±3 | 46±5 | 94±4 |
| Non Hodgkin Lymphoma T | 100 | 14±2 | 46±6 | 94±5 |
| Burkitt Lymphoma | 100 | 15±3 | 46±5 | 94±5 |
| Hodgkin lymphoma | 100 | 15±2 | 47±4 | 95±4 |
| Myeloma | 100 | 16±3 | 47±4 | 94±5 |

**Table 6. Percentage of inhibition of cell cultures treated with Vestipitant in relation to the percentage of cells that presented immunostaining for NK1 receptor.**

| **Type of Tumor** | **Number of cases.** | **Group 1 (expression in less than 20% of the cells).** | **Group 2 (expression in 20-80% of the cells).** | **Group 3 (expression in more than 80% of the cells).** |
|---|---|---|---|---|
| Gastric carcinoma | 100 | 11±3 | 48±3 | 92±3 |
| Colon carcinoma | 100 | 12±4 | 45±3 | 91±2 |
| Pancreatic carcinoma | 100 | 11±3 | 45±2 | 91±3 |
| Renal cell carcinoma | 100 | 10±4 | 45±3 | 93±6 |
| Breast carcinoma | 100 | 12±3 | 46±3 | 93±4 |
| Ovarian carcinoma | 100 | 14±4 | 47±4 | 95±5 |
| Endometrium carcinoma | 100 | 15±3 | 44±3 | 93±6 |
| Carcinoma of the uterine cervix | 100 | 17±4 | 46±3 | 94±4 |
| Lung carcinoma (non small cell type) | 100 | 15±2 | 45±2 | 93±4 |
| Lung carcinoma (small cell type) | 100 | 16±3 | 46±3 | 93±3 |
| Thyroid carcinoma (folliculary type) | 100 | 16±3 | 45±3 | 95±5 |
| Thyroid carcinoma (papillary type) | 100 | 15±3 | 47±5 | 96±4 |
| Urinary bladder carcinoma | 100 | 15±3 | 47±4 | 92±5 |
| Prostate carcinoma | 100 | 17±4 | 46±3 | 92±5 |
| Glioma | 100 | 15±4 | 45±4 | 92±4 |
| Fibrosarcoma | 100 | 16±3 | 45±3 | 91±3 |
| Malignant Fibrous Histiocytoma. | 100 | 15±4 | 46±4 | 91±5 |
| Ewing Sarcoma | 100 | 16±3 | 46±4 | 90±6 |
| Melanoma | 100 | 16±3 | 45±4 | 92±6 |
| Neuroblastoma | 100 | 17±2 | 46±3 | 93±3 |
| Leukemia B | 100 | 16±5 | 47±5 | 92±5 |
| Leukemia T | 100 | 17±4 | 46±3 | 94±4 |
| Non Hodgkin Lymphoma B | 100 | 14±3 | 45±5 | 93±5 |
| Non Hodgkin Lymphoma T | 100 | 13±4 | 46±4 | 93±6 |
| Burkitt Lymphoma | 100 | 12±3 | 45±3 | 92±4 |
| Hodgkin lymphoma | 100 | 14±3 | 46±3 | 92±5 |
| Myeloma | 100 | 15±2 | 45±4 | 93±6 |

**Table 7. Percentage of inhibition of cell cultures treated with antibody against NK1 receptor, in relation to the percentage of cells that presented immunostaining for NK1 receptor.**

| **Type of Tumor** | **Number of cases.** | **Group 1 (expression in less than 20% of the cells).** | **Group 2 (expression in 20-80% of the cells).** | **Group 3 (expression in more than 80% of the cells).** |
|---|---|---|---|---|
| Gastric carcinoma | 100 | 21±4 | 51±4 | 95±5 |
| Colon carcinoma | 100 | 22±4 | 53±4 | 94±4 |
| Pancreatic carcinoma | 100 | 23±3 | 53±4 | 96±5 |
| Renal cell carcinoma | 100 | 22±4 | 52±5 | 94±5 |
| Breast carcinoma | 100 | 22±4 | 54±4 | 96±5 |
| Ovarian carcinoma | 100 | 24±4 | 55±5 | 97±6 |
| Endometrium carcinoma | 100 | 22±5 | 53±3 | 96±5 |
| Carcinoma of the uterine cervix | 100 | 24±3 | 54±4 | 95±5 |
| Lung carcinoma (non small cell type) | 100 | 23±4 | 55±5 | 94±3 |
| Lung carcinoma (small cell type) | 100 | 24±4 | 56±3 | 95±4 |
| Thyroid carcinoma (folliculary type) | 100 | 25±2 | 54±3 | 96±4 |
| Thyroid carcinoma (papillary type) | 100 | 22±4 | 53±5 | 94±3 |
| Urinary bladder carcinoma | 100 | 23±5 | 54±3 | 95±5 |
| Prostate carcinoma | 100 | 23±4 | 57±4 | 96±4 |
| Glioma | 100 | 24±5 | 54±3 | 97±4 |
| Fibrosarcoma | 100 | 23±4 | 55±5 | 97±5 |
| Malignant Fibrous Histiocytoma. | 100 | 22±4 | 56±3 | 96±6 |
| Ewing Sarcoma | 100 | 23±4 | 54±3 | 95±4 |
| Melanoma | 100 | 24±5 | 55±5 | 96±5 |
| Neuroblastoma | 100 | 22±4 | 54±4 | 95±4 |
| Leukemia B | 100 | 23±3 | 55±4 | 95±5 |
| Leukemia T | 100 | 24±2 | 56±5 | 96±6 |
| Non Hodgkin Lymphoma B | 100 | 23±3 | 54±4 | 96±4 |
| Non Hodgkin Lymphoma T | 100 | 23±4 | 55±3 | 96±5 |
| Burkitt Lymphoma | 100 | 24±4 | 54±4 | 97±4 |
| Hodgkin lymphoma | 100 | 22±4 | 55±3 | 97±5 |
| Myeloma | 100 | 23±3 | 56±6 | 96±6 |

**Table 8. Percentage of inhibition of cell cultures treated with Aprepitant in relation to the intensity of the immunostaining for NK1 receptor.**

| **Tumor type (histologic type).** | **Control** | **Intensity grade 1** | **Intensity grade 2** | **Intensity grade 3** |
|---|---|---|---|---|
| Gastric carcinoma | 100 | 25±6 | 57±5 | 96±4 |
| Colon carcinoma | 100 | 26±6 | 56±6 | 95±5 |
| Pancreatic carcinoma | 100 | 27±7 | 56±5 | 94±3 |
| Renal cell carcinoma | 100 | 27±6 | 57±6 | 95±6 |
| Breast carcinoma | 100 | 28±6 | 58±5 | 95±7 |
| Ovarian carcinoma | 100 | 23±6 | 58±6 | 96±8 |
| Endometrium carcinoma | 100 | 25±7 | 59±5 | 94±5 |
| Carcinoma of the uterine cervix | 100 | 27±4 | 56±4 | 95±4 |
| Lung carcinoma (non small cell type) | 100 | 28±5 | 57±5 | 95±3 |
| Lung carcinoma (small cell type) | 100 | 29±6 | 56±6 | 96±4 |
| Thyroid carcinoma (folliculary type) | 100 | 26±6 | 57±5 | 95±5 |
| Thyroid carcinoma (papillary type) | 100 | 27±6 | 56±4 | 94±6 |
| Urinary bladder carcinoma | 100 | 26±5 | 57±3 | 96±6 |
| Prostate carcinoma | 100 | 27±5 | 58±6 | 93±7 |
| Glioma | 100 | 28±7 | 55±6 | 97±4 |
| Fibrosarcoma | 100 | 27±6 | 54±5 | 95±5 |
| Malignant Fibrous Histiocytoma. | 100 | 28±7 | 56±5 | 94±3 |
| Ewing Sarcoma | 100 | 27±6 | 57±4 | 95±6 |
| Melanoma | 100 | 26±4 | 55±5 | 96±7 |
| Neuroblastoma | 100 | 27±6 | 57±6 | 97±5 |
| Leukemia B | 100 | 28±5 | 54±5 | 95±6 |
| Leukemia T | 100 | 27±7 | 53±5 | 96±5 |
| Non Hodgkin Lymphoma B | 100 | 27±5 | 55±5 | 97±6 |
| Non Hodgkin Lymphoma T | 100 | 26±5 | 55±3 | 92±3 |
| Burkitt Lymphoma | 100 | 27±5 | 55±3 | 92±5 |
| Hodgkin lymphoma | 100 | 26±5 | 55±3 | 92±6 |
| Myeloma | 100 | 28±5 | 55±3 | 92±5 |

**Table 9. Percentage of inhibition of cell cultures treated with Casopitant in relation to the intensity of the immunostaining for NK1 receptor.**

| **Tumor type (histologic type).** | **Control** | **Intensity grade 1** | **Intensity grade 2** | **Intensity grade 3** |
|---|---|---|---|---|
| Gastric carcinoma | 100 | 31±3 | 53±3 | 91±3 |
| Colon carcinoma | 100 | 32±4 | 54±4 | 92±2 |
| Pancreatic carcinoma | 100 | 33±5 | 53±3 | 93±1 |
| Renal cell carcinoma | 100 | 34±2 | 55±5 | 91±4 |
| Breast carcinoma | 100 | 35±3 | 56±6 | 92±2 |
| Ovarian carcinoma | 100 | 33±4 | 54±4 | 91±3 |
| Endometrium carcinoma | 100 | 34±5 | 55±5 | 94±4 |
| Carcinoma of the uterine cervix | 100 | 33±3 | 53±3 | 92±2 |
| Lung carcinoma (non small cell type) | 100 | 32±4 | 54±4 | 93±3 |
| Lung carcinoma (small cell type) | 100 | 33±2 | 55±3 | 94±4 |
| Thyroid carcinoma (folliculary type) | 100 | 34±3 | 54±5 | 93±2 |
| Thyroid carcinoma (papillary type) | 100 | 35±4 | 55±6 | 92±3 |
| Urinary bladder carcinoma | 100 | 34±4 | 56±4 | 93±4 |
| Prostate carcinoma | 100 | 33±5 | 53±3 | 94±2 |
| Glioma | 100 | 36±3 | 54±5 | 91±3 |
| Fibrosarcoma | 100 | 35±6 | 55±3 | 92±4 |
| Malignant Fibrous Histiocytoma. | 100 | 32±5 | 53±4 | 93±2 |
| Ewing Sarcoma | 100 | 33±4 | 54±3 | 92±4 |
| Melanoma | 100 | 34±3 | 52±4 | 93±3 |
| Neuroblastoma | 100 | 35±4 | 55±5 | 94±4 |
| Leukemia B | 100 | 36±3 | 52±3 | 92±5 |
| Leukemia T | 100 | 34±2 | 56±4 | 93±3 |
| Non Hodgkin Lymphoma B | 100 | 32±3 | 55±5 | 94±4 |
| Non Hodgkin Lymphoma T | 100 | 36±5 | 54±5 | 92±2 |
| Burkitt Lymphoma | 100 | 37±5 | 55±3 | 93±3 |
| Hodgkin lymphoma | 100 | 34±5 | 54±4 | 92±4 |
| Myeloma | 100 | 35±3 | 55±3 | 93±3 |

**Table 10. Percentage of inhibition of cell cultures treated with Vestipitant in relation to the intensity of the immunostaining for NK1 receptor.**

| **Tumor type (histologic type).** | **Control** | **Intensity grade 1** | **Intensity grade 2** | **Intensity grade 3** |
|---|---|---|---|---|
| Gastric carcinoma | 100 | 19±4 | 49±4 | 92±4 |
| Colon carcinoma | 100 | 18±5 | 46±4 | 91±5 |
| Pancreatic carcinoma | 100 | 21±4 | 47±5 | 91±5 |
| Renal cell carcinoma | 100 | 21±3 | 46±4 | 93±4 |
| Breast carcinoma | 100 | 19±4 | 45±3 | 93±3 |
| Ovarian carcinoma | 100 | 15±3 | 46±2 | 95±4 |
| Endometrium carcinoma | 100 | 18±2 | 47±4 | 93±5 |
| Carcinoma of the uterine cervix | 100 | 19±3 | 48±3 | 94±3 |
| Lung carcinoma (non small cell type) | 100 | 17±2 | 49±5 | 93±5 |
| Lung carcinoma (small cell type) | 100 | 18±3 | 45±3 | 93±3 |
| Thyroid carcinoma (folliculary type) | 100 | 19±4 | 46±4 | 95±3 |
| Thyroid carcinoma (papillary type) | 100 | 16±2 | 47±5 | 96±5 |
| Urinary bladder carcinoma | 100 | 17±3 | 46±3 | 92±3 |
| Prostate carcinoma | 100 | 18±4 | 47±4 | 92±4 |
| Glioma | 100 | 19±3 | 48±5 | 92±3 |
| Fibrosarcoma | 100 | 18±2 | 47±3 | 91±2 |
| Malignant Fibrous Histiocytoma. | 100 | 17±3 | 48±4 | 91±5 |
| Ewing Sarcoma | 100 | 16±4 | 47±3 | 90±6 |
| Melanoma | 100 | 15±3 | 48±4 | 92±4 |
| Neuroblastoma | 100 | 16±1 | 49±5 | 93±4 |
| Leukemia B | 100 | 17±2 | 47±3 | 92±4 |
| Leukemia T | 100 | 15±3 | 48±4 | 94±5 |
| Non Hodgkin Lymphoma B | 100 | 16±4 | 47±5 | 93±4 |
| Non Hodgkin Lymphoma T | 100 | 17±2 | 48±4 | 93±5 |
| Burkitt Lymphoma | 100 | 15±3 | 47±5 | 92±6 |
| Hodgkin lymphoma | 100 | 16±4 | 48±3 | 92±4 |
| Myeloma | 100 | 17±2 | 47±4 | 93±5 |

**Table 11. Percentage of inhibition of cell cultures treated with antibody against NK1 receptor in relation to the intensity of the immunostaining for NK1 receptor.**

| **Tumor type (histologic type).** | **Control** | **Intensity grade 1** | **Intensity grade 2** | **Intensity grade 3** |
|---|---|---|---|---|
| Gastric carcinoma | 100 | 24±3 | 53±4 | 96±4 |
| Colon carcinoma | 100 | 23±3 | 52±4 | 96±3 |
| Pancreatic carcinoma | 100 | 22±4 | 54±4 | 93±4 |
| Renal cell carcinoma | 100 | 23±5 | 53±5 | 97±3 |
| Breast carcinoma | 100 | 24±3 | 54±4 | 96±4 |
| Ovarian carcinoma | 100 | 23±4 | 54±5 | 96±4 |
| Endometrium carcinoma | 100 | 24±3 | 54±3 | 97±6 |
| Carcinoma of the uterine cervix | 100 | 25±4 | 53±4 | 96±4 |
| Lung carcinoma (non small cell type) | 100 | 24±3 | 55±5 | 97±4 |
| Lung carcinoma (small cell type) | 100 | 25±3 | 54±3 | 94±3 |
| Thyroid carcinoma (folliculary type) | 100 | 26±4 | 53±3 | 95±5 |
| Thyroid carcinoma (papillary type) | 100 | 24±3 | 56±5 | 94±4 |
| Urinary bladder carcinoma | 100 | 25±4 | 55±3 | 96±3 |
| Prostate carcinoma | 100 | 24±5 | 56±4 | 93±6 |
| Glioma | 100 | 26±4 | 55±3 | 95±4 |
| Fibrosarcoma | 100 | 24±6 | 54±5 | 94±5 |
| Malignant Fibrous Histiocytoma. | 100 | 25±4 | 55±3 | 95±4 |
| Ewing Sarcoma | 100 | 24±5 | 55±3 | 96±4 |
| Melanoma | 100 | 23±3 | 56±5 | 97±4 |
| Neuroblastoma | 100 | 25±4 | 53±4 | 93±5 |
| Leukemia B | 100 | 24±3 | 54±4 | 94±6 |
| Leukemia T | 100 | 25±2 | 55±5 | 95±4 |
| Non Hodgkin Lymphoma B | 100 | 26±5 | 55±4 | 96±5 |
| Non Hodgkin Lymphoma T | 100 | 27±3 | 56±3 | 95±4 |
| Burkitt Lymphoma | 100 | 25±4 | 55±4 | 94±6 |
| Hodgkin lymphoma | 100 | 24±3 | 56±3 | 95±4 |
| Myeloma | 100 | 25±4 | 56±6 | 95±5 |

### Example 2. Cancer cells without the mutation in K-ras gene (non-mutated K-ras or "wild type") present a more effective response to treatment with antagonists of the NK1 receptor.

To verify that cancer cells without mutation in K-ras gene (non-mutated K-ras or "wild type") showed a more effective response to treatment with antagonists of the NK1 receptor, human primary tumor cells were taken from patients' tumors, the presence of NK1 receptor was checked by western-blot and immunohistochemical techniques, and it was determined if they presented or not mutations in the K-ras gene ("wild-type" form). Consecutively it was placed the cancer cells in cultures in the presence of several antagonists of the NK1 receptor. The different types of tumors and the patients' characteristics are shown in Table 12.

Tumor cells were gathered from human primary cancer samples taken from voluntary patients, and the presence of NK1 receptor was determined using Western-Blot and immunohistochemistry and the cells were cultured as it is explained in Example 1, in the presence of several antagonists of the NK1 receptor, such as Aprepitant, Casopitant, Vestipitant and an antibody against such receptor. In each case the presence or not of mutations in K-ras gene was determined.

To determine the existence of mutations in the K-ras gene DNA from cancer tumor cells was extracted from samples obtained from the different cancer tumor cells, using DNA extraction kits from Takara (Madison, WI, US) or Ambion (Huntingdon, Cambridgeshire, United Kingdom), following the manufacturer's instructions, and as it has been previously published (Di Fiore F, et al. 2007). Consecutively it was analyzed by sequencing the exon 2 of the KRAS gene, , by means of PCR amplification from previously extracted cancer tumor DNA, using the following sense primer (SEQ ID NO. 15): 5'-AAGGCCTGCTGAAAATGACTG-3' and anti-sense primer (SEQ ID NO. 16): 5'-CAAAGAATGGTCCTGCACCAG-3'. After the purification using the Qiagen extraction kit, the PCR products were sequenced using the Big Dye Terminator Kit V3.1 (Applied Biosystems, Foster City, CA, U.S.) and an ABI Prism 377 or 3100 DNA sequencer (Applied Biosystems). The presence of a heterozygous mutation in the KRAS gene in cancer tumor cells of the cultures was defined as the appearance of a mutant peak of a height of at least a third of the wild type. All the sequence analysis were executed at least twice in two independent PCR.

**Table 12. Characteristics of patients from whom the samples for cell cultures were extracted and number of cases with mutated Kras and "wild type" (wild).**

| **Tumor type (histologic type)** | **Number of cases (total)** | **Sex (men /woman)** | **Age** | **Number of cases with Kras mutated** | **Number of cases with Kras non-mutated (wild type).** |
|---|---|---|---|---|---|
| Gastric carcinoma (Adenocarcinoma) | 8 | 3/5 | 51±4 | 4 | 4 |
| Colon carcinoma (Adenocarcinoma) | 8 | 4/4 | 54±3 | 4 | 4 |
| Pancreatic carcinoma (Adenocarcinoma) | 8 | 3/5 | 53±2 | 4 | 4 |
| Renal cell carcinoma(clear cell renal cell carcinoma) | 8 | 4/4 | 52±4 | 4 | 4 |
| Breast carcinoma (ductal adenocarcinoma) | 8 | 0/8 | 46±5 | 4 | 4 |
| Ovarian carcinoma(Adenocarcinoma) | 8 | 0/8 | 51±4 | 4 | 4 |
| Endometrium carcinoma | 8 | 0/8 | 63±3 | 4 | 4 |
| Carcinoma of the uterine cervix (Squamous cell carcinoma). | 8 | 0/8 | 34±2 | 4 | 4 |
| Lung carcinoma (small cell lung carcinoma) | 8 | 6/2 | 52±4 | 4 | 4 |
| Lung carcinoma (non small cell lung carcinoma) | 8 | 6/2 | 64±3 | 4 | 4 |
| Thyroid carcinoma (follicular carcinoma). | 8 | 2/6 | 45±5 | 4 | 4 |
| Thyroid carcinoma (papillary carcinoma). | 8 | 2/6 | 38±4 | 4 | 4 |
| Urinary bladder carcinoma (transitional cell carcinoma) | 8 | 6/2 | 62±5 | 4 | 4 |
| Prostate carcinoma | 8 | 8/0 | 71±3 | 4 | 4 |
| Glioma (astrocytoma) | 8 | 5/3 | 56±4 | 4 | 4 |
| Undifferentiated Pleomorphic Sarcoma (malignant fibrous histiocytoma grade 3) | 8 | 5/3 | 67±2 | 4 | 4 |
| Melanoma (Nodular Melanoma) | 8 | 3/5 | 58±3 | 4 | 4 |
| Non Hodgkin Lymphoma B (diffuse B cell lymphoma) | 8 | 4/4 | 54±4 | 4 | 4 |
| Non Hodgkin Lymphoma T (diffuse T cell lymphoma) | 8 | 4/4 | 33±3 | 4 | 4 |
| Burkitt Lymphoma | 8 | 4/4 | 24±4 | 4 | 4 |

An increase can be observed in the inhibition of cell proliferation in cancer cell cultures which do not carry the Kras mutation. In Tables 13 to 16 it is shown how in cultures treated with Aprepitant (Table 13), Casopitant (Table 14), Vestipitant (Table 15), and antibodies against NK1 receptor - obtained from Sigma-Aldrich, catalogue number S8305- (Table 16), a higher inhibition of cell proliferation takes place in those cancer cell cultures which do not present the Kras mutation. Therefore, in cancer cells obtained from tumors that do not present the Kras mutation, the response to the treatment with antagonists of the NK1 receptor is higher. In the experiments reflected in Tables 13 to 15, Aprepitant, Casopitant and Vestipitant were used at 100 micromolar concentration. The antibody against NK1 receptor (Table 16) was used at 1/50 concentration. The percentages of inhibition are expressed in percentage of variation in relation to control ± standard deviation.

**Table 13. Percentage of inhibition of cell cultures treated with Aprepitant, in relation to the presence or not of Kras mutations in cancer tumor cells.**

| **Tumor type (histologic type)** | **Control** | **Cases with Kras mutated** | **Cases with Kras non-mutated (wild type).** |
|---|---|---|---|
| Gastric carcinoma (Adenocarcinoma) | 100 | 24±3 | 94±4 |
| Colon carcinoma (Adenocarcinoma) | 100 | 23±4 | 94±5 |
| Pancreatic carcinoma(Adenocarcinoma) | 100 | 22±4 | 94±4 |
| Renal cell carcinoma (clear cell renal cell carcinoma) | 100 | 23±3 | 94±5 |
| Breast carcinoma (ductal adenocarcinoma) | 100 | 22±2 | 94±3 |
| Ovarian carcinoma (Adenocarcinoma) | 100 | 24±5 | 94±4 |
| Endometrium carcinoma | 100 | 25±4 | 94±3 |
| Carcinoma of the uterine cervix (Squamous cell carcinoma). | 100 | 23±3 | 94±5 |
| Lung carcinoma (small cell lung carcinoma) | 100 | 22±4 | 94±4 |
| Lung carcinoma (non small cell lung carcinoma) | 100 | 21±2 | 94±3 |
| Thyroid carcinoma (follicular carcinoma). | 100 | 26±3 | 94±6 |
| Thyroid carcinoma (papillary carcinoma). | 100 | 25±4 | 94±5 |
| Urinary bladder carcinoma (transitional cell carcinoma) | 100 | 26±5 | 94±4 |
| Prostate carcinoma | 100 | 24±3 | 94±3 |
| Glioma (astrocytoma) | 100 | 23±4 | 94±4 |
| Undifferentiated Pleomorphic Sarcoma (malignant fibrous histiocytoma grade 3). | 100 | 24±3 | 94±3 |
| Melanoma (Nodular Melanoma). | 100 | 26±4 | 94±4 |
| Non Hodgkin Lymphoma B (diffuse B cell lymphoma). | 100 | 27±5 | 94±3 |
| Non Hodgkin Lymphoma T (diffuse T cell lymphoma). | 100 | 25±4 | 94±4 |
| Burkitt Lymphoma | 100 | 24±3 | 94±5 |

**Table 14. Percentage of inhibition of cell cultures treated with Casopitant, in relation to the presence or not of Kras mutations in cancer tumor cells.**

| **Tumor type (histologic type)** | **Control** | **Cases with Kras mutated** | **Cases with Kras non-mutated (wild type).** |
|---|---|---|---|
| Gastric carcinoma (Adenocarcinoma) | 100 | 25±5 | 95±5 |
| Colon carcinoma (Adenocarcinoma) | 100 | 32±4 | 94±6 |
| Pancreatic carcinoma (Adenocarcinoma) | 100 | 27±3 | 93±4 |
| Renal cell carcinoma (clear cell renal cell carcinoma) | 100 | 27±4 | 94±3 |
| Breast carcinoma (ductal adenocarcinoma) | 100 | 28±6 | 96±4 |
| Ovarian carcinoma (Adenocarcinoma) | 100 | 29±4 | 97±6 |
| Endometrium carcinoma | 100 | 28±2 | 94±4 |
| Carcinoma of the uterine cervix (Squamous cell carcinoma). | 100 | 27±3 | 93±3 |
| Lung carcinoma (small cell lung carcinoma) | 100 | 26±4 | 94±2 |
| Lung carcinoma (non small cell lung carcinoma) | 100 | 25±5 | 95±5 |
| Thyroid carcinoma (follicular carcinoma). | 100 | 26±4 | 96±4 |
| Thyroid carcinoma (papillary carcinoma). | 100 | 24±5 | 94±5 |
| Urinary bladder carcinoma (transitional cell carcinoma) | 100 | 25±4 | 95±6 |
| Prostate carcinoma | 100 | 23±3 | 93±7 |
| Glioma (astrocytoma) | 100 | 26±4 | 94±6 |
| Undifferentiated Pleomorphic Sarcoma (malignant fibrous histiocytoma grade 3). | 100 | 26±5 | 95±5 |
| Melanoma (Nodular Melanoma). | 100 | 25±6 | 94±6 |
| Non Hodgkin Lymphoma B (diffuse B cell lymphoma). | 100 | 26±3 | 95±4 |
| Non Hodgkin Lymphoma T (diffuse T cell lymphoma). | 100 | 26±4 | 96±5 |
| Burkitt Lymphoma | 100 | 25±2 | 92±5 |

**Table 15. Percentage of inhibition of cell cultures treated with Vestipitant, in relation to the presence or not of Kras mutations in cancer tumor cells.**

| **Tumor type (histologic type)** | **Control** | **Cases with Kras mutated** | **Cases with Kras non-mutated (wild type).** |
|---|---|---|---|
| Gastric carcinoma (Adenocarcinoma) | 100 | 24±4 | 96±4 |
| Colon carcinoma (Adenocarcinoma) | 100 | 33±5 | 96±5 |
| Pancreatic carcinoma (Adenocarcinoma) | 100 | 24±4 | 97±4 |
| Renal cell carcinoma (clear cell renal cell carcinoma) | 100 | 25±5 | 95±6 |
| Breast carcinoma (ductal adenocarcinoma) | 100 | 26±4 | 97±5 |
| Ovarian carcinoma (Adenocarcinoma) | 100 | 27±5 | 96±6 |
| Endometrium carcinoma | 100 | 24±6 | 95±4 |
| Carcinoma of the uterine cervix (Squamous cell carcinoma). | 100 | 25±5 | 94±3 |
| Lung carcinoma (small cell lung carcinoma) | 100 | 23±4 | 95±4 |
| Lung carcinoma (non small cell lung carcinoma) | 100 | 24±5 | 96±6 |
| Thyroid carcinoma (follicular carcinoma). | 100 | 25±3 | 97±4 |
| Thyroid carcinoma (papillary carcinoma). | 100 | 26±4 | 94±5 |
| Urinary bladder carcinoma (transitional cell carcinoma) | 100 | 25±5 | 95±6 |
| Prostate carcinoma | 100 | 24±4 | 96±4 |
| Glioma (astrocytoma) | 100 | 25±3 | 97±5 |
| Undifferentiated Pleomorphic Sarcoma (malignant fibrous histiocytoma grade 3). | 100 | 26±4 | 95±6 |
| Melanoma (Nodular Melanoma) | 100 | 23±5 | 96±5 |
| Non Hodgkin Lymphoma B (diffuse B cell lymphoma). | 100 | 24±4 | 97±6 |
| Non Hodgkin Lymphoma T (diffuse T cell lymphoma). | 100 | 25±5 | 96±5 |
| Burkitt Lymphoma | 100 | 24±3 | 95±3 |

**Table 16. Percentage of inhibition of cell cultures treated with antibodies to the NK1 receptor, in relation to the presence or not of Kras mutations in cancer tumor cells.**

| **Tumor type (histologic type)** | **Control** | **Cases with Kras mutated** | **Cases with Kras non-mutated (wild type).** |
|---|---|---|---|
| Gastric carcinoma (Adenocarcinoma) | 100 | 24±6 | 94±6 |
| Colon carcinoma (Adenocarcinoma) | 100 | 29±7 | 93±4 |
| Pancreatic carcinoma (Adenocarcinoma) | 100 | 24±5 | 95±7 |
| Renal cell carcinoma (clear cell renal cell carcinoma) | 100 | 25±4 | 96±4 |
| Breast carcinoma (ductal adenocarcinoma) | 100 | 27±5 | 93±5 |
| Ovarian carcinoma (Adenocarcinoma) | 100 | 28±3 | 92±3 |
| Endometrium carcinoma | 100 | 25±2 | 93±4 |
| Carcinoma of the uterine cervix (Squamous cell carcinoma). | 100 | 23±4 | 94±6 |
| Lung carcinoma (small cell lung carcinoma) | 100 | 28±3 | 95±4 |
| Lung carcinoma (non small cell lung carcinoma) | 100 | 25±2 | 96±5 |
| Thyroid carcinoma (follicular carcinoma). | 100 | 23±3 | 97±4 |
| Thyroid carcinoma (papillary carcinoma). | 100 | 25±4 | 94±5 |
| Urinary bladder carcinoma (transcional cell carcinoma) | 100 | 27±2 | 95±3 |
| Prostate carcinoma | 100 | 28±3 | 96±4 |
| Glioma (astrocytoma) | 100 | 24±2 | 93±5 |
| Undifferentiated Pleomorphic Sarcoma (malignant fibrous histiocytoma grade 3). | 100 | 25±4 | 94±4 |
| Melanoma (Nodular Melanoma) | 100 | 26±3 | 95±3 |
| Non Hodgkin Lymphoma B (diffuse B cell lymphoma). | 100 | 23±2 | 93±4 |
| Non Hodgkin Lymphoma T (diffuse T cell lymphoma). | 100 | 24±4 | 94±5 |
| Burkitt Lymphoma | 100 | 26±3 | 95±4 |

### Example 3. Cancer cells presenting more activity in the MAP Kinases pathway present a more effective response to treatment with antagonists of the NK1 receptor.

To check that cancer cells having higher activity in the MAP Kinases pathway show a more effective response to the treatment with antagonists of the NK1 receptor, we obtained human primary tumor cells from patients' tumors, and the presence of NK1 receptor was checked by western-blot and immunohistochemical techniques, and the absence of mutations in K-ras ("wild type" form). Consecutively, the cells were placed in cultures in the presence of several antagonists of the NK1 receptor. The different types of tumors and the patients' characteristics are shown in Table 17.

Tumor cells were gathered from human primary cancer samples obtained from voluntary patients, the presence of NK1 receptor was determined using Western-Blot and immunohistochemistry and the cells were cultured as it is explained in Example 1, in the presence of several antagonists of the NK1 receptor, such as Aprepitant, Casopitant, Vestipitant an antibody against such receptor. In all cases it was checked the absence of mutations in Kras, following the method explained in Example 2. In each case it was determined the presence or absence of the downstream effector in the MAP Kinases ERK pathway. Also the presence of MEK was determined, with similar results.

To determine the presence of the ERK protein, it was performed a Western Blot technique, using as primary antibody: Phospho-p44/42 MAPK (Erk1/2) (Thr202/Tyr204) (D13.14.4E) XP^{®} Rabbit mAb (4370, Cell Signalling). To determine the presence of the MEK protein, it was performed the Western Blot technique, using as primary antibody Phospho-MEK1/2 (Ser217/221) (9121, Cell Signalling).

**Table 17. Characteristics of patients from whom the samples for cell cultures were extracted, and number of cases with absence or presence of ERK protein.**

| **Tumor type (histologic type)** | **Number of cases** | **Sex (men/woman)** | **Age** | **Number of cases with absense of ERK** | **Number of cases with presence of ERK** |
|---|---|---|---|---|---|
| Gastric carcinoma (Adenocarcinoma) | 8 | 4/4 | 52±5 | 4 | 4 |
| Colon carcinoma (Adenocarcinoma) | 8 | 3/5 | 52±4 | 4 | 4 |
| Pancreatic carcinoma (Adenocarcinoma) | 8 | 3/5 | 54±3 | 4 | 4 |
| Renal cell carcinoma (clear cell renal cell carcinoma) | 8 | 5/3 | 51±5 | 4 | 4 |
| Breast carcinoma (ductal adenocarcinoma) | 8 | 0/8 | 49±4 | 4 | 4 |
| Ovarian carcinoma(Adenocarcinoma) | 8 | 0/8 | 52±3 | 4 | 4 |
| Endometrium carcinoma | 8 | 0/8 | 62±4 | 4 | 4 |
| Carcinoma of the uterine cervix (Squamous cell carcinoma). | 8 | 0/8 | 35±5 | 4 | 4 |
| Lung carcinoma (small cell lung carcinoma) | 8 | 6/2 | 54±3 | 4 | 4 |
| Lung carcinoma (non small cell lung carcinoma) | 8 | 6/2 | 63±4 | 4 | 4 |
| Thyroid carcinoma (follicular carcinoma). | 8 | 2/6 | 44±4 | 4 | 4 |
| Thyroid carcinoma (papillary carcinoma). | 8 | 2/6 | 35±3 | 4 | 4 |
| Urinary bladder carcinoma (transitional cell carcinoma) | 8 | 6/2 | 66±4 | 4 | 4 |
| Prostate carcinoma | 8 | 8/0 | 71±4 | 4 | 4 |
| Glioma (astrocytoma) | 8 | 5/3 | 55±3 | 4 | 4 |
| Undifferentiated Pleomorphic Sarcoma (malignant fibrous histiocytoma grade 3). | 8 | 5/3 | 66±4 | 4 | 4 |
| Melanoma (Nodular Melanoma). | 8 | 3/5 | 54±4 | 4 | 4 |
| Non Hodgkin Lymphoma B (diffuse B cell lymphoma). | 8 | 4/4 | 53±5 | 4 | 4 |
| Non Hodgkin Lymphoma T (diffuse T cell lymphoma). | 8 | 4/4 | 35±4 | 4 | 4 |
| Burkitt Lymphoma | 8 | 4/4 | 26±5 | 4 | 4 |

It is appreciated an increase in the inhibition of cell proliferation in cancer cell cultures, which shows the presence of ERK in the efferent downstream MAP Kinases pathway. In Tables 18 to 21 it is shown how in cultures treated with Aprepitant (Table 18), Casopitant (Table 19), Vestipitant (Table 20), and antibodies against NK1 receptor - obtained from Sigma-Aldrich, catalogue number S8305- (Table 21), a higher inhibition of cell proliferation can be seen in those cancer cell cultures that show more presence of ERK. Therefore, in cancer cells obtained from tumors present ERK protein, which is an indication of the activation of the MAP Kinases pathway in these tumors, the response to treatment with antagonists of the NK1 receptor is higher. Similar results were found in the case of the MEK protein (higher response in cancer tumors presenting higher amount of MEK protein). In the experiments shown in Tables 18 to 20, Aprepitant, Casopitant and Vestipitant were used at 100 micromolar concentration. The antibody against NK1 receptor (Table 21) was used at 1/50 concentration. The percentages of inhibition are expressed in percentages of variation in relation to control ± standard deviation.

**Table 18. Percentage of inhibition of cell cultures treated with aprepitant, in relation to the absence or presence of ERK protein.**

| **Tumor type (histologic type)** | **Control** | **Number of cases without presence of ERK** | **Number of cases with presence of ERK** |
|---|---|---|---|
| Gastric carcinoma (Adenocarcinoma) | 100 | 23±7 | 96±5 |
| Colon carcinoma (Adenocarcinoma) | 100 | 24±7 | 95±4 |
| Pancreatic carcinoma (Adenocarcinoma) | 100 | 23±6 | 94±5 |
| Renal cell carcinoma (clear cell renal cell carcinoma) | 100 | 24±4 | 97±5 |
| Breast carcinoma (ductal adenocarcinoma) | 100 | 25±5 | 93±5 |
| Ovarian carcinoma (Adenocarcinoma) | 100 | 26±7 | 92±5 |
| Endometrium carcinoma | 100 | 24±4 | 93±4 |
| Carcinoma of the uterine cervix (Squamous cell carcinoma). | 100 | 25±5 | 94±5 |
| Lung carcinoma (small cell lung carcinoma) | 100 | 26±6 | 95±3 |
| Lung carcinoma (non small cell lung carcinoma) | 100 | 27±4 | 96±4 |
| Thyroid carcinoma (follicular carcinoma). | 100 | 24±5 | 95±6 |
| Thyroid carcinoma (papillary carcinoma). | 100 | 23±3 | 93±7 |
| Urinary bladder carcinoma (transitional cell carcinoma) | 100 | 25±4 | 94±5 |
| Prostate carcinoma | 100 | 26±5 | 95±6 |
| Glioma (astrocytoma) | 100 | 27±6 | 93±4 |
| Undifferentiated Pleomorphic Sarcoma (malignant fibrous histiocytoma grade 3). | 100 | 24±4 | 94±5 |
| Melanoma (Nodular Melanoma). | 100 | 25±5 | 96±6 |
| Non Hodgkin Lymphoma B (diffuse B cell | 100 | 26±6 | 97±3 |
| lymphoma). | | | |
| Non Hodgkin Lymphoma T (diffuse T cell lymphoma). | 100 | 23±3 | 94±4 |
| Burkitt Lymphoma | 100 | 24±4 | 93±5 |

**Table 19. Percentage of inhibition of cell cultures treated with casopitant, in relation to the absence or presence of ERK protein.**

| **Tumor type (histologic type)** | **Control** | **Number of cases without presence of ERK** | **Number of cases with presence of ERK** |
|---|---|---|---|
| Gastric carcinoma (Adenocarcinoma) | 100 | 25±6 | 96±4 |
| Colon carcinoma (Adenocarcinoma) | 100 | 24±3 | 95±3 |
| Pancreatic carcinoma (Adenocarcinoma) | 100 | 23±4 | 94±2 |
| Renal cell carcinoma (clear cell renal cell carcinoma) | 100 | 27±3 | 94±3 |
| Breast carcinoma (ductal adenocarcinoma) | 100 | 26±4 | 93±4 |
| Ovarian carcinoma (Adenocarcinoma) | 100 | 28±5 | 92±6 |
| Endometrium carcinoma | 100 | 29±2 | 93±5 |
| Carcinoma of the uterine cervix (Squamous cell carcinoma). | 100 | 28±3 | 93±4 |
| Lung carcinoma (small cell lung carcinoma) | 100 | 29±4 | 94±5 |
| Lung carcinoma (non small cell lung carcinoma) | 100 | 27±5 | 95±7 |
| Thyroid carcinoma (follicular carcinoma). | 100 | 26±4 | 96±6 |
| Thyroid carcinoma (papillary carcinoma). | 100 | 28±3 | 97±4 |
| Urinary bladder carcinoma (transitional cell carcinoma) | 100 | 29±4 | 98±2 |
| Prostate carcinoma | 100 | 27±4 | 94±5 |
| Glioma (astrocytoma) | 100 | 28±3 | 93±4 |
| Undifferentiated Pleomorphic Sarcoma (malignant fibrous histiocytoma grade 3). | 100 | 26±4 | 95±3 |
| Melanoma (Nodular Melanoma). | 100 | 27±5 | 96±5 |
| Non Hodgkin Lymphoma B (diffuse B cell lymphoma). | 100 | 28±4 | 97±5 |
| Non Hodgkin Lymphoma T (diffuse T cell lymphoma). | 100 | 29±5 | 98±4 |
| Burkitt Lymphoma | 100 | 28±6 | 97±5 |

**Table 20. Percentage of inhibition of cell cultures treated with vestipitant, in relation to the absence or presence of ERK protein.**

| **Tumor type (histologic type)** | **Control** | **Number of cases without presence of ERK** | **Number of cases with presence of ERK** |
|---|---|---|---|
| Gastric carcinoma (Adenocarcinoma) | 100 | 24±4 | 95±3 |
| Colon carcinoma (Adenocarcinoma) | 100 | 23± | 94±3 |
| Pancreatic carcinoma (Adenocarcinoma) | 100 | 24±34 | 93±4 |
| Renal cell carcinoma (clear cell renal cell carcinoma) | 100 | 25±2 | 92±2 |
| Breast carcinoma (ductal adenocarcinoma) | 100 | 26±4 | 94±3 |
| Ovarian carcinoma (Adenocarcinoma) | 100 | 24±2 | 95±5 |
| Endometrium carcinoma | 100 | 28±5 | 93±3 |
| Carcinoma of the uterine cervix (Squamous cell carcinoma). | 100 | 29±3 | 94±4 |
| Lung carcinoma(small cell lung carcinoma) | 100 | 27±4 | 96±6 |
| Lung carcinoma (non small cell lung carcinoma) | 100 | 26±2 | 95±5 |
| Thyroid carcinoma (follicular carcinoma). | 100 | 25±3 | 94±4 |
| Thyroid carcinoma (papillary carcinoma). | 100 | 26±4 | 96±3 |
| Urinary bladder carcinoma (transitional cell carcinoma) | 100 | 24±5 | 97±5 |
| Prostate carcinoma | 100 | 22±3 | 94±4 |
| Glioma (astrocytoma) | 100 | 27±4 | 95±3 |
| Undifferentiated Pleomorphic Sarcoma (malignant fibrous histiocytoma grade 3). | 100 | 26±2 | 93±4 |
| Melanoma (Nodular Melanoma). | 100 | 25±3 | 94±5 |
| Non Hodgkin Lymphoma B (diffuse B cell lymphoma). | 100 | 24±4 | 96±3 |
| Non Hodgkin Lymphoma T (diffuse T cell lymphoma). | 100 | 25±2 | 92±4 |
| Burkitt Lymphoma | 100 | 26±4 | 93±5 |

**Table 21. Percentage of inhibition of cell cultures treated with antibodies against NK1 receptor, in relation to the absence or presence of ERK protein.**

| **Tumor type (histologic type)** | **Control** | **Number of cases without presence of ERK** | **Number of cases with presence of ERK** |
|---|---|---|---|
| Gastric carcinoma (Adenocarcinoma) | 100 | 27±5 | 91±4 |
| Colon carcinoma (Adenocarcinoma) | 100 | 28±5 | 95±5 |
| Pancreatic carcinoma (Adenocarcinoma) | 100 | 94±4 | 94±3 |
| Renal cell carcinoma (clear cell renal cell carcinoma) | 100 | 32±6 | 95±6 |
| Breast carcinoma (ductal adenocarcinoma) | 100 | 29±5 | 94±7 |
| Ovarian carcinoma (Adenocarcinoma) | 100 | 24±7 | 96±5 |
| Endometrium carcinoma | 100 | 29±6 | 95±4 |
| Carcinoma of the uterine cervix (Squamous cell carcinoma). | 100 | 28±5 | 96±6 |
| Lung carcinoma (small cell lung carcinoma) | 100 | 27±6 | 94±4 |
| Lung carcinoma (non small cell lung carcinoma) | 100 | 29±7 | 95±5 |
| Thyroid carcinoma (follicular carcinoma). | 100 | 26±5 | 96±3 |
| Thyroid carcinoma (papillary carcinoma). | 100 | 27±6 | 89±4 |
| Urinary bladder carcinoma (transitional cell carcinoma) | 100 | 28±4 | 88±5 |
| Prostate carcinoma | 100 | 27±3 | 90±4 |
| Glioma (astrocytoma) | 100 | 28±4 | 89±5 |
| Undifferentiated Pleomorphic Sarcoma (malignant fibrous histiocytoma grade 3). | 100 | 27±5 | 90±4 |
| Melanoma (Nodular Melanoma). | 100 | 28±3 | 90±5 |
| Non Hodgkin Lymphoma B (diffuse B cell lymphoma). | 100 | 29±4 | 98±6 |
| Non Hodgkin Lymphoma T (diffuse T cell lymphoma). | 100 | 27±5 | 97±4 |
| Burkitt Lymphoma | 100 | 28±3 | 96±5 |

### Example 4. Cancer cells presenting higher activity in the PI3 kinases pathway have a more effective response to the treatment with antagonists of the NK1 receptor.

To check that cancer cells having higher activity in the PI3 Kinases pathway show a more effective response to treatment with antagonists of the NK1 receptor, human primary tumor cells were obtained from patients' tumors, and it was confirmed that they presented NK1 receptor by western-blot and immunohistochemistry techniques, and that they did not carried out mutations in K-ras ("wild type" form). Afterwards, the cells were cultured in presence of several antagonists of the NK1 receptor. The different tumors and characteristics of patients are shown in Table 22.

Tumor cells were gathered from human primary cancer samples obtained from voluntary patients, the presence of NK1 receptor was determined using Western-Blot and immunohistochemistry and the cells were cultured as it is explained in Example 1, in the presence of several antagonists of the NK1 receptor, such as Aprepitant, Casopitant, Vestipitant, and an antibody against such receptor. In each case absence of the mutation in the Kras was checked, following the method explained in Example 2. In each case it was determined the presence or absence of the AKT protein effector downstream in the PI3 Kinases pathway.

To determine the presence of the AKT protein in the different cultures Western Blot technique was performed (following the description in example 1-Western Blot section), but using as primary antibody with reference: Akt (pan) (11E7) Rabbit mAb (4685, Cell Signalling).

**Table 22. Characteristics of patients from whom samples for cell cultures were obtained and number of cases with absence or presence of AKT protein.**

| **Tumor type (histologic type)** | **Number of cases** | **Sex (men/ woman)** | **Age** | **Number of cases with absense of AKT** | **Number of cases with presence of AKT** |
|---|---|---|---|---|---|
| Gastric carcinoma (Adenocarcinoma) | 8 | 4/4 | 51±4 | 4 | 4 |
| Colon carcinoma (Adenocarcinoma) | 8 | 3/5 | 50±3 | 4 | 4 |
| Pancreatic carcinoma (Adenocarcinoma) | 8 | 3/5 | 55±4 | 4 | 4 |
| Renal cell carcinoma (clear cell renal cell carcinoma) | 8 | 5/3 | 50±3 | 4 | 4 |
| Breast carcinoma (ductal adenocarcinoma) | 8 | 0/8 | 48±2 | 4 | 4 |
| Ovarian carcinoma(Adenocarcinoma) | 8 | 0/8 | 52±2 | 4 | 4 |
| Endometrium carcinoma | 8 | 0/8 | 61±3 | 4 | 4 |
| Carcinoma of the uterine cervix (Squamous cell carcinoma). | 8 | 0/8 | 36±4 | 4 | 4 |
| Lung carcinoma (small cell lung carcinoma) | 8 | 6/2 | 53±5 | 4 | 4 |
| Lung carcinoma (non small cell lung carcinoma) | 8 | 6/2 | 62±3 | 4 | 4 |
| Thyroid carcinoma (follicular carcinoma) | 8 | 2/6 | 43±2 | 4 | 4 |
| Thyroid carcinoma (papillary carcinoma) | 8 | 2/6 | 36±4 | 4 | 4 |
| Urinary bladder carcinoma | 8 | 6/2 | 63±5 | 4 | 4 |
| (transitional cell carcinoma) | | | | | |
| Prostate carcinoma | 8 | 8/0 | 70±3 | 4 | 4 |
| Glioma (astrocytoma) | 8 | 5/3 | 58±3 | 4 | 4 |
| Undifferentiated Pleomorphic Sarcoma (malignant fibrous histiocytoma grade 3). | 8 | 5/3 | 65±3 | 4 | 4 |
| Melanoma (Nodular Melanoma). | 8 | 3/5 | 57±3 | 4 | 4 |
| Non Hodgkin Lymphoma B (diffuse B cell lymphoma). | 8 | 4/4 | 54±4 | 4 | 4 |
| Non Hodgkin Lymphoma T (diffuse T cell lymphoma). | 8 | 4/4 | 33±5 | 4 | 4 |
| Burkitt Lymphoma | 8 | 4/4 | 28±3 | 4 | 4 |

It is appreciated an increase in the inhibition of cell proliferation in cancer cell cultures, which shows the presence of the efferent AKT protein of the PI3 Kinase pathway. In Tables 23 to 26 it is shown how in cultures treated with Aprepitant (Table 23), Casopitant (Table 24), Vestipitant (Table 25), and NK1 receptor antibodies - obtained from Sigma-Aldrich, catalogue number S8305-(Table 26), a higher inhibition of cell proliferation takes place in those cancer cell cultures showing more presence of AKT. Therefore, in cancer cells obtaining from tumors presenting AKT protein, which is an indication of the activation of the PI3 Kinases pathway in these tumors, the response to treatment with antagonists to NK1 receptor is higher. In the experiments shown in Tables 23 to 25, Aprepitant, Casopitant, and Vestipitant were used at 100 micromolar concentration. The antibody against NK1 receptor (Table 26) was used at 1/50 concentration. The percentages of inhibition are expressed in percentage of variation in relation to control ± standard deviation.

**Table 23. Percentage of inhibition of cell cultures treated with aprepitant, in relation to the absence or presence of AKT protein.**

| **Tumor type (histologic type)** | **Control** | **Number of cases without presence of AKT** | **Number of cases with presence of AKT** |
|---|---|---|---|
| Gastric carcinoma (Adenocarcinoma) | 100 | 24±6 | 92±4 |
| Colon carcinoma (Adenocarcinoma) | 100 | 25±3 | 93±3 |
| Pancreatic carcinoma (Adenocarcinoma) | 100 | 24±4 | 92±2 |
| Renal cell carcinoma (clear cell renal cell carcinoma) | 100 | 25±3 | 93±3 |
| Breast carcinoma (ductal adenocarcinoma) | 100 | 26±5 | 94±4 |
| Ovarian carcinoma (Adenocarcinoma) | 100 | 27±4 | 93±3 |
| Endometrium carcinoma | 100 | 25±3 | 95±5 |
| Carcinoma of the uterine cervix (Squamous cell carcinoma). | 100 | 24±4 | 94±4 |
| Lung carcinoma (small cell lung carcinoma) | 100 | 25±3 | 96±3 |
| Lung carcinoma (non small cell lung carcinoma) | 100 | 26±4 | 94±4 |
| Thyroid carcinoma (follicular carcinoma). | 100 | 24±3 | 97±3 |
| Thyroid carcinoma (papillary carcinoma). | 100 | 25±4 | 92±4 |
| Urinary bladder carcinoma (transitional cell carcinoma) | 100 | 23±5 | 94±2 |
| Prostate carcinoma | 100 | 24±4 | 97±6 |
| Glioma (astrocytoma) | 100 | 25±5 | 96±5 |
| Undifferentiated Pleomorphic Sarcoma (malignant fibrous histiocytoma grade 3). | 100 | 26±4 | 95±4 |
| Melanoma (Nodular Melanoma) | 100 | 25±5 | 94±5 |
| Non Hodgkin Lymphoma B (diffuse B cell lymphoma). | 100 | 24±6 | 95±3 |
| Non Hodgkin Lymphoma T (diffuse T cell lymphoma). | 100 | 25±3 | 93±5 |
| Burkitt Lymphoma | 100 | 26±4 | 94±4 |

**Table 24. Percentage of inhibition of cell cultures treated with casopitant, in relation to the absence or presence of AKT protein.**

| **Tumor type (histologic type)** | **Control** | **Number of cases without presence of AKT** | **Number of cases with presence of AKT** |
|---|---|---|---|
| Gastric carcinoma (Adenocarcinoma) | 100 | 26±5 | 95±3 |
| Colon carcinoma (Adenocarcinoma) | 100 | 24±5 | 96±4 |
| Pancreatic carcinoma (Adenocarcinoma) | 100 | 23±4 | 95±3 |
| Renal cell carcinoma (clear cell renal cell carcinoma) | 100 | 24±3 | 96±5 |
| Breast carcinoma (ductal adenocarcinoma) | 100 | 23±4 | 95±4 |
| Ovarian carcinoma (Adenocarcinoma) | 100 | 24±3 | 94±5 |
| Endometrium carcinoma | 100 | 23±4 | 95±46 |
| Carcinoma of the uterine cervix (Squamous cell carcinoma). | 100 | 24±3 | 97±4 |
| Lung carcinoma (small cell lung carcinoma) | 100 | 23±4 | 96±5 |
| Lung carcinoma (non small cell lung carcinoma) | 100 | 25±6 | 95±4 |
| Thyroid carcinoma (follicular carcinoma). | 100 | 24±5 | 96±5 |
| Thyroid carcinoma (papillary carcinoma). | 100 | 26±2 | 95±4 |
| Urinary bladder carcinoma (transitional cell carcinoma) | 100 | 24±3 | 94±3 |
| Prostate carcinoma | 100 | 23±4 | 95±3 |
| Glioma (astrocytoma) | 100 | 27±3 | 96±4 |
| Undifferentiated Pleomorphic Sarcoma (malignant fibrous histiocytoma grade 3). | 100 | 25±3 | 95±3 |
| Melanoma (Nodular Melanoma). | 100 | 23±4 | 94±4 |
| Non Hodgkin Lymphoma B (diffuse B cell lymphoma). | 100 | 24±5 | 95±5 |
| Non Hodgkin Lymphoma T (diffuse T cell lymphoma). | 100 | 23±3 | 98±4 |
| Burkitt Lymphoma | 100 | 24±4 | 96±3 |

**Table 25. Percentage of inhibition of cell cultures treated with vestipitant, in relation to the absence or presence of AKT protein.**

| **Tumor type (histologic type)** | **Control** | **Number of cases without presence of AKT** | **Number of cases with presence of AKT** |
|---|---|---|---|
| Gastric carcinoma (Adenocarcinoma) | 100 | 25±5 | 94±4 |
| Colon carcinoma (Adenocarcinoma) | 100 | 24±3 | 93±5 |
| Pancreatic carcinoma (Adenocarcinoma) | 100 | 23±5 | 94±4 |
| Renal cell carcinoma (clear cell renal cell carcinoma) | 100 | 23±3 | 96±5 |
| Breast carcinoma (ductal adenocarcinoma) | 100 | 22±5 | 95±4 |
| Ovarian carcinoma (Adenocarcinoma) | 100 | 23±6 | 94±6 |
| Endometrium carcinoma | 100 | 24±4 | 95±5 |
| Carcinoma of the uterine cervix (Squamous cell carcinoma). | 100 | 25±5 | 93±4 |
| Lung carcinoma (small cell lung carcinoma) | 100 | 23±4 | 94±5 |
| Lung carcinoma (non small cell lung carcinoma) | 100 | 24±3 | 95±4 |
| Thyroid carcinoma (follicular carcinoma). | 100 | 23±4 | 94±5 |
| Thyroid carcinoma (papillary carcinoma). | 100 | 25±5 | 96±4 |
| Urinary bladder carcinoma (transitional cell carcinoma) | 100 | 24±4 | 95±5 |
| Prostate carcinoma | 100 | 26±3 | 94±4 |
| Glioma (astrocytoma) | 100 | 23±4 | 95±5 |
| Undifferentiated Pleomorphic Sarcoma (malignant fibrous histiocytoma grade 3). | 100 | 27±3 | 94±6 |
| Melanoma (Nodular Melanoma). | 100 | 25±4 | 93±5 |
| Non Hodgkin Lymphoma B (diffuse B cell lymphoma). | 100 | 24±3 | 95±6 |
| Non Hodgkin Lymphoma T (diffuse T cell lymphoma). | 100 | 25±5 | 96±5 |
| Burkitt Lymphoma | 100 | 24±4 | 95±6 |

**Table 26. Percentage of inhibition of cell cultures treated with NK1 receptor antibodies, in relation to the absence or presence of AKT protein.**

| **Tumor type (histologic type)** | **Control** | **Number of cases without presence of AKT** | **Number of cases with presence of AKT** |
|---|---|---|---|
| Gastric carcinoma (Adenocarcinoma) | 100 | 24±4 | 92±5 |
| Colon carcinoma (Adenocarcinoma) | 100 | 23±3 | 94±3 |
| Pancreatic carcinoma (Adenocarcinoma) | 100 | 94±4 | 95±4 |
| Renal cell carcinoma (clear cell renal cell carcinoma) | 100 | 34±5 | 94±3 |
| Breast carcinoma (ductal adenocarcinoma) | 100 | 25±4 | 93±5 |
| Ovarian carcinoma (Adenocarcinoma) | 100 | 24±6 | 95±3 |
| Endometrium carcinoma | 100 | 23±5 | 95±4 |
| Carcinoma of the uterine cervix (Squamous cell carcinoma). | 100 | 23±4 | 96±5 |
| Lung carcinoma (small cell lung carcinoma) | 100 | 24±5 | 94±4 |
| Lung carcinoma (non small cell lung carcinoma) | 100 | 25±6 | 95±5 |
| Thyroid carcinoma(follicular carcinoma). | 100 | 24±5 | 96±4 |
| Thyroid carcinoma (papillary carcinoma). | 100 | 24±6 | 89±5 |
| Urinary bladder carcinoma (transitional cell carcinoma) | 100 | 25±5 | 91±6 |
| Prostate carcinoma | 100 | 26±6 | 90±5 |
| Glioma (astrocytoma) | 100 | 28±4 | 89±4 |
| Undifferentiated Pleomorphic Sarcoma (malignant fibrous histiocytoma grade 3). | 100 | 25±5 | 90±3 |
| Melanoma (Nodular Melanoma). | 100 | 25±3 | 97±4 |
| Non Hodgkin Lymphoma B (diffuse B cell lymphoma). | 100 | 24±4 | 95±5 |
| Non Hodgkin Lymphoma T (diffuse T cell lymphoma). | 100 | 25±3 | 94±4 |
| Burkitt Lymphoma | 100 | 25±2 | 95±6 |

### Example 5. Cancer cells presenting higher amount of the truncated form of NK1 receptor have a more effective response to the treatment with antagonists of the NK1 receptor.

To check that cancer cells having higher amount of the truncated form of NK1 receptor have a more effective response to treatment with antagonists of the NK1 receptor, human primary tumor cells were obtained from patients' tumors, the presence of NK1 receptor was checked using western-blot and immunohistochemistry. The presence of the truncated form of NK1 receptor was determined and then cells were cultured in presence of several antagonists of the NK1 receptor. The different tumors and characteristics of patients are shown in Table 26.

Tumor cells were gathered from human primary cancer samples obtained from voluntary patients, the presence of NK1 receptor was determined using Western-Blot and immunohistochemistry and the cells were cultured as it is explained in Example 1, in the presence of several antagonists of the NK1 receptor, such as Aprepitant, Casopitant, Vestipitant, and an antibody against such receptor. In each case the presence of the truncated form of the NK1 receptor was determined.

To determine the presence of the truncated form of the gene of NK1 receptor PCR technique was performed (Polymerase Chain Reaction). The extraction of RNA, amplifications of PCR, cycle conditions and quantitative analysis were executed as it has been previously described. The specific primers were the following: For the full-length NK1R receptor (NM_001058.3; forward (SEQ ID NO: 17)-: 5'-AACCCCATCATCTACTGCTGC-3 ' and reverse- (SEQ ID NO:18): 5'-ATTTCCAGCCCCTCATAGTCG-3') and for the truncated NK1receptor (NM_015727.2; forward (SEQ ID NO: 19): 5'-GGGCCACAAGACCATCTACA-3 and reverse (SEQ ID NO;20): 5'-AAGTTAGCTGCAGTCCCCAC-3').

Cases were distributed in two groups:
- Group A -low expression of the truncated form of NK1 receptor- for those cases wherein the expression of the truncated form of NK1 receptor was less than double the expression of the full length form of such receptor.
- Group B -high expression of the truncated form of NK1 receptor- for those cases wherein the expression of the truncated form of the NK1 receptor was higher than double the expression of the full length form of such receptor.

**Table 27. Characteristics of patients from whom the samples for cell cultures were extracted, and number of cases with low expression of the truncated form of NK1 receptor (Group A) and high expression of the truncated form of NK1 receptor (Group B).**

| **Tumor type (histologic type)** | **Number of cases** | **Sex (men / woman)** | **Age** | **Number of cases with low expression of the truncated form of NK1 receptor (Group A)** | **Number of cases with high expression of the truncated form of NK1 receptor (Group B)** |
|---|---|---|---|---|---|
| Gastric carcinoma (Adenocarcinoma) | 8 | 4/4 | 50±4 | 4 | 4 |
| Colon carcinoma (Adenocarcinoma) | 8 | 4/4 | 53±3 | 4 | 4 |
| Pancreatic carcinoma (Adenocarcinoma) | 8 | 3/5 | 55±4 | 4 | 4 |
| Renal cell carcinoma (clear cell renal cell carcinoma) | 8 | 5/3 | 56±3 | 4 | 4 |
| Breast carcinoma (ductal adenocarcinoma) | 8 | 0/8 | 43±2 | 4 | 4 |
| Ovarian carcinoma(Adenocarcinoma) | 8 | 0/8 | 51±2 | 4 | 4 |
| Endometrium carcinoma | 8 | 0/8 | 62±3 | 4 | 4 |
| Carcinoma of the uterine cervix (Squamous cell carcinoma) | 8 | 0/8 | 33±4 | 4 | 4 |
| Lung carcinoma (small cell lung carcinoma) | 8 | 5/3 | 54±5 | 4 | 4 |
| Lung carcinoma (non small cell lung carcinoma) | 8 | 6/2 | 63±3 | 4 | 4 |
| Thyroid carcinoma (follicular carcinoma) | 8 | 2/6 | 45±2 | 4 | 4 |
| Thyroid carcinoma (papillary carcinoma) | 8 | 2/6 | 33±4 | 4 | 4 |
| Urinary bladder carcinoma (transitional cell carcinoma) | 8 | 6/2 | 64±5 | 4 | 4 |
| Prostate carcinoma | 8 | 8/0 | 70±3 | 4 | 4 |
| Glioma (astrocytoma) | 8 | 4/2 | 50±3 | 4 | 4 |
| Undifferentiated Pleomorphic Sarcoma (malignant fibrous histiocytoma grade 3). | 8 | 5/3 | 62±3 | 4 | 4 |
| Melanoma (Nodular Melanoma). | 8 | 3/5 | 54±3 | 4 | 4 |
| Non Hodgkin Lymphoma B (diffuse B cell lymphoma). | 8 | 4/4 | 55±4 | 4 | 4 |
| Non Hodgkin Lymphoma T (diffuse T cell lymphoma). | 8 | 4/4 | 32±5 | 4 | 4 |
| Burkitt Lymphoma | 8 | 4/4 | 22±3 | 4 | 4 |

It is appreciated an increase in the inhibition of cell proliferation in cancer cell cultures, which shows a high expression of the truncated form of NK1 receptor. In Tables 28 to 31 it is shown how in cultures treated with Aprepitant (Table 28), Casopitant (Table 29), Vestipitant (Table 30), and NK1 receptor antibodies -obtained from Sigma-Aldrich, catalogue number S8305- (Table 31), a higher inhibition of cell proliferation takes place in those cancer cell cultures that show more expression of the truncated form of NK1 receptor. Therefore, in cancer cells obtained from tumors presenting a high expression of the truncated form of NK1 receptor, which is an indication of the activation of the PI3 Kinases pathway in those tumors, the response to treatment with antagonists of the NK1 receptor is higher. In the experiments shown in Tables 28 to 30, Aprepitant, Casopitant and Vestipitant were used at a 100 micromolar concentration. The antibody against NK1 receptor (Table 31) was used at 1/50 concentration. The percentages of inhibition are expressed in percentage of variation in relation to the control ± standard deviation.

**Table 28. Percentage of inhibition of cell cultures treated with aprepitant, in relation to the existence of low expression of the truncated form of NK1 receptor (Group A) or high expression of the truncated form of the NK1 receptor (Group B).**

| **Tumor type (histologic type)** | **Control** | **Cases with low expression of the truncated form of NK1 receptor (Group A)** | **Cases with high expression of the truncated form of NK1 receptor (Group B)** |
|---|---|---|---|
| Gastric carcinoma (Adenocarcinoma) | 100 | 25±5 | 94±3 |
| Colon carcinoma (Adenocarcinoma) | 100 | 26±4 | 93±4 |
| Pancreatic carcinoma (Adenocarcinoma) | 100 | 23±4 | 94±5 |
| Renal cell carcinoma (clear cell renal cell carcinoma) | 100 | 26±3 | 93±3 |
| Breast carcinoma (ductal adenocarcinoma) | 100 | 27±5 | 94±4 |
| Ovarian carcinoma (Adenocarcinoma) | 100 | 29±3 | 95±5 |
| Endometrium carcinoma | 100 | 26±5 | 96±3 |
| Carcinoma of the uterine cervix (Squamous cell carcinoma). | 100 | 26±5 | 94±4 |
| Lung carcinoma (small cell lung carcinoma) | 100 | 24±4 | 96±3 |
| Lung carcinoma (non small cell lung carcinoma) | 100 | 25±3 | 94±4 |
| Thyroid carcinoma (follicular carcinoma). | 100 | 26±4 | 95±5 |
| Thyroid carcinoma (papillary carcinoma). | 100 | 27±5 | 96±4 |
| Urinary bladder carcinoma (transitional cell carcinoma) | 100 | 25±6 | 95±4 |
| Prostate carcinoma | 100 | 24±6 | 94±5 |
| Glioma (astrocytoma) | 100 | 27±5 | 95±5 |
| Undifferentiated Pleomorphic Sarcoma (malignant fibrous histiocytoma grade 3). | 100 | 27±6 | 96±6 |
| Melanoma (Nodular Melanoma). | 100 | 25±7 | 96±5 |
| Non Hodgkin Lymphoma B (diffuse B cell lymphoma). | 100 | 23±4 | 97±7 |
| Non Hodgkin Lymphoma T (diffuse T cell lymphoma). | 100 | 26±5 | 98±6 |
| Burkitt Lymphoma | 100 | 27±6 | 99±5 |

**Table 29. Percentage of inhibition of cell cultures treated with casopitant, in relation to the existence of low expression of the truncated form of NK1 receptor (Group A) or high expression of the truncated form of the NK1 receptor (Group B).**

| **Tumor type (histologic type)** | **Control** | **Cases with low expression of the truncated form of NK1 receptor (Group A)** | **Cases with high expression of the truncated form of NK1 receptor (Group B)** |
|---|---|---|---|
| Gastric carcinoma (Adenocarcinoma) | 100 | 22±4 | 95±3 |
| Colon carcinoma (Adenocarcinoma) | 100 | 22±3 | 94±2 |
| Pancreatic carcinoma (Adenocarcinoma) | 100 | 23±4 | 95±4 |
| Renal cell carcinoma (clear cell renal cell carcinoma) | 100 | 24±5 | 94±5 |
| Breast carcinoma (ductal adenocarcinoma) | 100 | 23±3 | 95±3 |
| Ovarian carcinoma (Adenocarcinoma) | 100 | 25±4 | 95±6 |
| Endometrium carcinoma | 100 | 26±3 | 94±5 |
| Carcinoma of the uterine cervix (Squamous cell carcinoma). | 100 | 25±5 | 93±6 |
| Lung carcinoma (small cell lung carcinoma) | 100 | 24±5 | 94±5 |
| Lung carcinoma (non small cell lung carcinoma) | 100 | 25±4 | 95±6 |
| Thyroid carcinoma (follicular carcinoma). | 100 | 23±3 | 93±4 |
| Thyroid carcinoma (papillary carcinoma). | 100 | 24±5 | 94±5 |
| Urinary bladder carcinoma (transitional cell carcinoma) | 100 | 25±4 | 95±7 |
| Prostate carcinoma | 100 | 23±6 | 94±4 |
| Glioma (astrocytoma) | 100 | 24±5 | 95±7 |
| Undifferentiated Pleomorphic Sarcoma (malignant fibrous histiocytoma grade 3). | 100 | 25±4 | 94±5 |
| Melanoma (Nodular Melanoma). | 100 | 23±5 | 96±6 |
| Non Hodgkin Lymphoma B (diffuse B cell lymphoma). | 100 | 24±3 | 97±4 |
| Non Hodgkin Lymphoma T (diffuse T cell lymphoma). | 100 | 23±4 | 98±3 |
| Burkitt Lymphoma | 100 | 24±5 | 93±3 |

**Table 30. Percentage of inhibition of cell cultures treated with vestipitant, in relation to the existence of low expression of the truncated form of NK1 receptor (Group A) or high expression of the truncated form of the NK1 receptor (Group B).**

| **Tumor type (histologic type)** | **Control** | **Cases with low expression of the truncated form of NK1 receptor (Group A)** | **Cases with high expression of the truncated form of NK1 receptor (Group B)** |
|---|---|---|---|
| Gastric carcinoma (Adenocarcinoma) | 100 | 24±4 | 94±4 |
| Colon carcinoma (Adenocarcinoma) | 100 | 23±3 | 93±3 |
| Pancreatic carcinoma (Adenocarcinoma) | 100 | 24±4 | 94±4 |
| Renal cell carcinoma (clear cell renal cell carcinoma) | 100 | 25±5 | 95±5 |
| Breast carcinoma (ductal adenocarcinoma) | 100 | 24±3 | 93±3 |
| Ovarian carcinoma (Adenocarcinoma) | 100 | 25±4 | 94±6 |
| Endometrium carcinoma | 100 | 26±3 | 95±5 |
| Carcinoma of the uterine cervix (Squamous cell carcinoma). | 100 | 24±5 | 96±6 |
| Lung carcinoma (small cell lung carcinoma) | 100 | 25±5 | 95±6 |
| Lung carcinoma (non small cell lung carcinoma) | 100 | 26±4 | 94±4 |
| Thyroid carcinoma (follicular carcinoma). | 100 | 27±3 | 95±5 |
| Thyroid carcinoma (papillary carcinoma). | 100 | 26±5 | 96±6 |
| Urinary bladder carcinoma (transitional cell carcinoma) | 100 | 25±4 | 94±5 |
| Prostate carcinoma | 100 | 24±6 | 95±6 |
| Glioma (astrocytoma) | 100 | 25±5 | 94±5 |
| Undifferentiated Pleomorphic Sarcoma (malignant fibrous histiocytoma grade 3). | 100 | 26±4 | 95±3 |
| Melanoma (Nodular Melanoma). | 100 | 24±5 | 96±4 |
| Non Hodgkin Lymphoma B (diffuse B cell lymphoma). | 100 | 23±5 | 98±5 |
| Non Hodgkin Lymphoma T (diffuse T cell lymphoma). | 100 | 23±4 | 98±4 |
| Burkitt Lymphoma | 100 | 24±5 | 93±5 |

**Table 31. Percentage of inhibition of cell cultures treated with NK1 receptor antibody, in relation to the existence of low expression of the truncated form of NK1 receptor (Group A) or high expression of the truncated form of the NK1 receptor (Group B).**

| **Tumor type (histologic type)** | **Control** | **Cases with low expression of the truncated form of NK1 receptor (Group A)** | **Cases with high expression of the truncated form of NK1 receptor (Group B)** |
|---|---|---|---|
| Gastric carcinoma (Adenocarcinoma) | 100 | 22±4 | 96±4 |
| Colon carcinoma (Adenocarcinoma) | 100 | 24±3 | 94±3 |
| Pancreatic carcinoma (Adenocarcinoma) | 100 | 23±4 | 95±4 |
| Renal cell carcinoma (clear cell renal cell carcinoma) | 100 | 24±5 | 93±5 |
| Breast carcinoma (ductal adenocarcinoma) | 100 | 24±3 | 94±3 |
| Ovarian carcinoma (Adenocarcinoma) | 100 | 25±4 | 95±6 |
| Endometrium carcinoma | 100 | 26±3 | 94±5 |
| Carcinoma of the uterine cervix (Squamous cell carcinoma). | 100 | 24±5 | 95±6 |
| Lung carcinoma (small cell lung carcinoma) | 100 | 25±5 | 96±6 |
| Lung carcinoma (non small cell lung carcinoma) | 100 | 26±4 | 95±4 |
| Thyroid carcinoma (follicular carcinoma). | 100 | 24±3 | 94±5 |
| Thyroid carcinoma (papillary carcinoma). | 100 | 25±5 | 95±6 |
| Urinary bladder carcinoma (transitional cell carcinoma) | 100 | 26±4 | 96±5 |
| Prostate carcinoma | 100 | 27±6 | 95±6 |
| Glioma (astrocytoma) | 100 | 24±5 | 96±5 |
| Undifferentiated Pleomorphic Sarcoma (malignant fibrous histiocytoma grade 3). | 100 | 25±4 | 95±3 |
| Melanoma (Nodular Melanoma). | 100 | 26±5 | 96±4 |
| Non Hodgkin Lymphoma B (diffuse B cell lymphoma). | 100 | 24±5 | 95±5 |
| Non Hodgkin Lymphoma T (diffuse T cell lymphoma). | 100 | 25±4 | 95±4 |
| Burkitt Lymphoma | 100 | 26±5 | 96±5 |

### Example 6. Cancer cells presenting amplification at the level of the gene encoding for the NK1 receptor show a more effective response to the treatment with antagonists of the NK1 receptor.

To check that cancer cells having amplification at the level of the gene that encodes for the NK1 receptor show a more effective response to treatment with antagonists of the NK1 receptor, human primary tumor cells were obtained from patients' tumors, and the presence of NK1 receptor was tested by western-blot and immunohistochemical techniques. The presence or absence of the amplification of the gene encoding for the NK1 receptor was determined, and then the cells were cultured in the presence of several antagonists of the NK1 receptor. The different types of tumors and the patients' characteristics are shown in Table 32.

Tumor cells were gathered from human primary cancer samples which were obtained from voluntary patients, the presence of NK1 receptor was determined using Western-Blot and immunohistochemistry and the cells were cultured as it is explained in Example 1, in the presence of several antagonists of the NK1 receptor, such as Aprepitant, Casopitant, Vestipitant an antibody against such receptor. For each case, the presence or absence of amplification of the gene encoding for the NK1 receptor was determined.

In order to determine the presence of the amplification (increase in the number of copies) of the gene encoding the NK1 receptor (TAC1R), it was used the comparative genomic hybridization technique (CGH Array). Firstly genomic DNA was extracted from each case. Later, it was performed the marking and hybridization of the DNA of each sample together with DNA of a commercial reference without genomic alterations (from masculine or feminine sex, depending on the gender of the patient from whom the samples were obtained). The Array is Sureprint G3 Human CGH Microarray from the company Agilent was used, with an average separation between probes of 5.3Kb and 4.6Kb for the genes RefSeq. The reading of the microarray was done with the DNA Microarray Scanner G2565CA from Agilent, at a resolution of 3 µm. The analysis of the results was performed with the software Cytogenomics v 2.0.6.0 from Agilent with ADM-2: 6.0; Centralization: ON (Threshold: 6.0, Bin size: 10); Fuzzy zero: ON; Aberration filters: ON (minProbes=5 and minAvgAbsLopRatio= 0,5); Feature Level Filters : ON. The genomic coordinates are coincident with the NCBI36genome (hg18). Cases were distributed in two groups: Group I for those cases without the amplification of the gene encoding the NK1 receptor (locus 2p13.1-p12), and Group II for those cases with amplification of the gene encoding the NK1receptor (locus 2p13.1-p12).

**Table 32. Characteristics of patients from whom the samples for cell cultures were obtained and number of cases of Group I without amplification of the gene that encodes for the NK1 receptor (locus 2p13.1-p12), and Group II with amplification of the gene that encodes for the NK1 receptor (locus 2p13.1-p12).**

| **Tumor type (histologic type)** | **Number of cases** | **Sex (men / woman)** | **Age** | **Number of cases without amplification of the gene that encodes for the NK1 receptor** | **Number of cases with amplification of the gene that encodes for the NK1 receptor** |
|---|---|---|---|---|---|
| Gastric carcinoma (Adenocarcinoma) | 8 | 4/4 | 54±4 | 4 | 4 |
| Colon carcinoma (Adenocarcinoma) | 8 | 4/4 | 54±3 | 4 | 4 |
| Pancreatic carcinoma (Adenocarcinoma) | 8 | 4/4 | 52±4 | 4 | 4 |
| Renal cell carcinoma (clear cell renal cell carcinoma) | 8 | 4/4 | 51±3 | 4 | 4 |
| Breast carcinoma (ductal adenocarcinoma) | 8 | 0/8 | 43±2 | 4 | 4 |
| Ovarian carcinoma(Adenocarcinoma) | 8 | 0/8 | 53±2 | 4 | 4 |
| Endometrium carcinoma | 8 | 0/8 | 62±3 | 4 | 4 |
| Carcinoma of the uterine cervix (Squamous cell carcinoma). | 8 | 0/8 | 33±4 | 4 | 4 |
| Lung carcinoma (small cell lung carcinoma) | 8 | 5/3 | 53±5 | 4 | 4 |
| Lung carcinoma (non small cell lung carcinoma) | 8 | 6/2 | 63±3 | 4 | 4 |
| Thyroid carcinoma (follicular carcinoma). | 8 | 2/6 | 45±2 | 4 | 4 |
| Thyroid carcinoma (papillary carcinoma). | 8 | 2/6 | 33±4 | 4 | 4 |
| Urinary bladder carcinoma (transitional cell carcinoma) | 8 | 6/2 | 64±5 | 4 | 4 |
| Prostate carcinoma | 8 | 8/0 | 71±4 | 4 | 4 |
| Glioma (astrocytoma) | 8 | 4/2 | 50±3 | 4 | 4 |
| Undifferentiated Pleomorphic Sarcoma (malignant fibrous histiocytoma grade 3). | 8 | 5/3 | 62±2 | 4 | 4 |
| Melanoma (Nodular Melanoma). | 8 | 3/5 | 53±5 | 4 | 4 |
| Non Hodgkin Lymphoma B (diffuse B cell lymphoma). | 8 | 4/4 | 55±4 | 4 | 4 |
| Non Hodgkin Lymphoma T (diffuse T cell lymphoma). | 8 | 4/4 | 32±5 | 4 | 4 |
| Burkitt Lymphoma | 8 | 4/4 | 22±3 | 4 | 4 |

It is shown an increase in the inhibition of cell proliferation in cancer cell cultures which show the amplification of the gene encoding the NK1 receptor. In Tables 33 to 36 it is shown how in cultures treated with Aprepitant (Table 33), Casopitant (Table 34), Vestipitant (Table 35), and NK1 receptor antibodies -obtained from Sigma-Aldrich, catalogue number S8305- (Table 36), a higher inhibition of cell proliferation takes place in those cancer cell cultures which show amplification in the genomic region encoding the NK1 receptor. Therefore, in cancer cells obtained from tumors presenting amplification in the genomic region encoding the NK1 receptor, the response to the treatment with antagonists of NK1 is higher (more effective). In the experiments shown in Tables 33 to 35 Aprepitant, Casopitant and Vestipitant were used at 100 micromolar concentration. The antibody against NK1 receptor (Table 36) was used at 1/50 concentration. The percentages of inhibition are expressed in percentages of variation in relation to control ± standard deviation.

**Table 33. Percentage of inhibition of cell cultures treated with aprepitant, in relation to the absence of amplification of the gene encoding for the NK1 receptor (Group I) or the presence of amplification at the level of the gene for the NK1 receptor (Group II).**

| **Tumor type (histologic type)** | **Control** | **Cases without amplification of the gene that encodes for the NK1 receptor** | **Cases with amplification of the gene that encodes for the NK1 receptor** |
|---|---|---|---|
| Gastric carcinoma (Adenocarcinoma) | 100 | 24±6 | 96±6 |
| Colon carcinoma (Adenocarcinoma) | 100 | 23±4 | 94±4 |
| Pancreatic carcinoma (Adenocarcinoma) | 100 | 25±5 | 95±5 |
| Renal cell carcinoma (clear cell renal cell carcinoma) | 100 | 26±3 | 97±7 |
| Breast carcinoma (ductal adenocarcinoma) | 100 | 24±4 | 99±4 |
| Ovarian carcinoma(Adenocarcinoma) | 100 | 23±6 | 92±5 |
| Endometrium carcinoma | 100 | 24±5 | 94±5 |
| Carcinoma of the uterine cervix (Squamous cell carcinoma). | 100 | 25±4 | 94±6 |
| Lung carcinoma (small cell lung carcinoma) | 100 | 26±5 | 93±4 |
| Lung carcinoma (non small cell lung carcinoma) | 100 | 27±3 | 95±5 |
| Thyroid carcinoma (follicular carcinoma). | 100 | 24±4 | 96±6 |
| Thyroid carcinoma (papillary carcinoma). | 100 | 25±5 | 97±4 |
| Urinary bladder carcinoma (transitional cell carcinoma) | 100 | 23±4 | 97±3 |
| Prostate carcinoma | 100 | 25±4 | 94±6 |
| Glioma (astrocytoma) | 100 | 25±5 | 95±5 |
| Undifferentiated Pleomorphic Sarcoma | 100 | 26±7 | 96±4 |
| (malignant fibrous histiocytoma grade 3). | | | |
| Melanoma (Nodular Melanoma). | 100 | 24±4 | 97±5 |
| Non Hodgkin Lymphoma B (diffuse B cell lymphoma). | 100 | 25±5 | 93±6 |
| Non Hodgkin Lymphoma T (diffuse T cell lymphoma). | 100 | 26±3 | 94±7 |
| Burkitt Lymphoma | 100 | 25±5 | 95±4 |

**Table 34. Percentage of inhibition of cell cultures treated with casopitant, in relation to the absence of amplification of the gene encoding for the NK1 receptor (Group I) or the presence of amplification at the level of the gene encoding for the NK1 receptor (Group II).**

| **Tumor type (histologic type)** | **Control** | **Cases without amplification of the gene that encodes for the NK1 receptor** | **Cases with amplification of the gene that encodes for the NK1 receptor** |
|---|---|---|---|
| Gastric carcinoma (Adenocarcinoma) | 100 | 23±5 | 95±5 |
| Colon carcinoma (Adenocarcinoma) | 100 | 24±4 | 96±4 |
| Pancreatic carcinoma (Adenocarcinoma) | 100 | 26±6 | 97±5 |
| Renal cell carcinoma (clear cell renal cell carcinoma) | 100 | 27±7 | 93±6 |
| Breast carcinoma (ductal adenocarcinoma) | 100 | 23±8 | 94±7 |
| Ovarian carcinoma(Adenocarcinoma) | 100 | 24±5 | 95±4 |
| Endometrium carcinoma | 100 | 24±4 | 96±5 |
| Carcinoma of the uterine cervix (Squamous cell carcinoma). | 100 | 23±6 | 94±6 |
| Lung carcinoma (small cell lung carcinoma) | 100 | 24±4 | 95±4 |
| Lung carcinoma (non small cell lung carcinoma) | 100 | 26±6 | 96±5 |
| Thyroid carcinoma (follicular carcinoma). | 100 | 26±5 | 94±6 |
| Thyroid carcinoma (papillary carcinoma). | 100 | 24±7 | 96±5 |
| Urinary bladder carcinoma (transitional cell carcinoma) | 100 | 26±4 | 97±3 |
| Prostate carcinoma | 100 | 27±5 | 96±5 |
| Glioma (astrocytoma) | 100 | 24±6 | 95±4 |
| Undifferentiated Pleomorphic Sarcoma (malignant fibrous histiocytoma grade 3). | 100 | 25±7 | 96±6 |
| Melanoma (Nodular Melanoma). | 100 | 26±4 | 97±5 |
| Non Hodgkin Lymphoma B (diffuse B cell lymphoma). | 100 | 27±5 | 95±7 |
| Non Hodgkin Lymphoma T (diffuse T cell lymphoma). | 100 | 28±7 | 95±5 |
| Burkitt Lymphoma | 100 | 24±5 | 93±6 |

**Table 35. Percentage of inhibition of cell cultures treated with Vestipitant, in relation to the absence of amplification of the gene encoding for the NK1 receptor (Group I) or the presence of amplification at the level of the gene encoding for the NK1 receptor (Group II).**

| **Tumor type (histologic type)** | **Control** | **Cases without amplification of the gene that encodes for the NK1 receptor** | **Cases with amplification of the gene that encodes for the NK1 receptor** |
|---|---|---|---|
| Gastric carcinoma (Adenocarcinoma) | 100 | 25±5 | 94±4 |
| Colon carcinoma (Adenocarcinoma) | 100 | 24±4 | 93±3 |
| Pancreatic carcinoma (Adenocarcinoma) | 100 | 25±3 | 93±4 |
| Renal cell carcinoma (clear cell renal cell carcinoma) | 100 | 27±4 | 94±5 |
| Breast carcinoma (ductal adenocarcinoma) | 100 | 24±3 | 94±6 |
| Ovarian carcinoma(Adenocarcinoma) | 100 | 25±4 | 96±5 |
| Endometrium carcinoma | 100 | 23±5 | 95±5 |
| Carcinoma of the uterine cervix (Squamous cell carcinoma). | 100 | 26±6 | 94±6 |
| Lung carcinoma (small cell lung carcinoma) | 100 | 25±3 | 94±4 |
| Lung carcinoma (non small cell lung carcinoma) | 100 | 24±4 | 93±5 |
| Thyroid carcinoma (follicular carcinoma). | 100 | 25±5 | 94±6 |
| Thyroid carcinoma (papillary carcinoma). | 100 | 26±3 | 97±4 |
| Urinary bladder carcinoma (transitional cell carcinoma) | 100 | 24±5 | 95±5 |
| Prostate carcinoma | 100 | 25±6 | 96±6 |
| Glioma (astrocytoma) | 100 | 26±5 | 94±7 |
| Undifferentiated Pleomorphic Sarcoma (malignant fibrous histiocytoma grade 3). | 100 | 27±4 | 95±7 |
| Melanoma (Nodular Melanoma). | 100 | 24±6 | 96±5 |
| Non Hodgkin Lymphoma B (diffuse B cell lymphoma). | 100 | 27±5 | 94±6 |
| Non Hodgkin Lymphoma T (diffuse T cell lymphoma). | 100 | 29±3 | 95±4 |
| Burkitt Lymphoma | 100 | 23±5 | 97±5 |

**Table 36. Percentage of inhibition of cell cultures treated with NK1 receptor antibodies in relation to the absence of amplification of the gene encoding for the NK1 receptor (Group I) or the presence of amplification at the level of the gene encoding for the NK1 receptor (Group II).**

| **Tumor type (histologic type)** | **Control** | **Cases without amplification of the gene that encodes for the NK1 receptor** | **Cases with amplification of the gene that encodes for the NK1 receptor** |
|---|---|---|---|
| Gastric carcinoma (Adenocarcinoma) | 100 | 25±4 | 95±6 |
| Colon carcinoma (Adenocarcinoma) | 100 | 24±5 | 96±5 |
| Pancreatic carcinoma (Adenocarcinoma) | 100 | 25±6 | 96±6 |
| Renal cell carcinoma (clear cell renal cell carcinoma) | 100 | 27±7 | 97±7 |
| Breast carcinoma (ductal adenocarcinoma) | 100 | 26±5 | 96±4 |
| Ovarian carcinoma (Adenocarcinoma) | 100 | 24±6 | 95±5 |
| Endometrium carcinoma | 100 | 28±4 | 96±6 |
| Carcinoma of the uterine cervix (Squamous cell carcinoma). | 100 | 27±3 | 94±6 |
| Lung carcinoma (small cell lung carcinoma) | 100 | 26±4 | 95±3 |
| Lung carcinoma (non small cell lung carcinoma) | 100 | 25±5 | 97±4 |
| Thyroid carcinoma (follicular carcinoma). | 100 | 26±4 | 95±6 |
| Thyroid carcinoma (papillary carcinoma). | 100 | 27±5 | 96±7 |
| Urinary bladder carcinoma (transitional cell carcinoma) | 100 | 24±4 | 94±5 |
| Prostate carcinoma | 100 | 25±3 | 95±6 |
| Glioma (astrocytoma) | 100 | 25±5 | 95±5 |
| Undifferentiated Pleomorphic Sarcoma (malignant fibrous histiocytoma grade 3). | 100 | 25±6 | 96±4 |
| Melanoma (Nodular Melanoma). | 100 | 26±5 | 97±5 |
| Non Hodgkin Lymphoma B (diffuse B cell lymphoma). | 100 | 26±4 | 94±4 |
| Non Hodgkin Lymphoma T (diffuse T cell lymphoma). | 100 | 24±3 | 95±6 |
| Burkitt Lymphoma | 100 | 25±5 | 96±7 |

### Example 7. Cancer cell presenting a constitutively activated form of NK1 receptor, show a more effective response to treatment with antagonists of the NK1 receptor.

To verify that cancer cells presenting a constitutively activated form of NK1 receptor show a more effective response to treatment with antagonists of the NK1 receptor, human primary tumor cells were obtained from patients' tumors, and the presence of NK1 receptor was checked using western-blot and immunohistochemistry. It was determined the increase or not in proliferation with the exposition to substance P (NK1 receptor agonist), so that for the cell cultures which grew independently from the exposition to substance P, it was considered that they have constitutively activated forms of such receptor, because a mitogenic signal independently from the presence of the agonist was transmitted. It was checked that all tumor cells presented integrity in the MAP Kinases and PI3 Kinases pathways, as wel as in the K-ras gene, and the protein derived from it.

Tumor cells were gathered from human primary cancer samples obtained from voluntary patients, the presence of NK1 receptor was determined using Western-Blot and immunohistochemistry and the cells were cultured as it is explained in Example 1, in the presence of the agonist substance P. Group I was constituted by cells with little proliferating response to the exposition to substance P (constitutively activated receptor, independently from its coupling to the agonist) and Group II was constituted by cells with higher proliferating response to the exposition to the agonist of such receptor substance P (receptor with normal response to stimulus with agonist).

Table 37 shows how higher inhibition of tumor cell growth takes place with the treatment with the antagonist Aprepitant in cancer tumors in the group with little proliferating response when exposed to substance P (in presence of the constitutively activated form of NK1 receptor). Similar results can be observed with Vestipitant, Casopitant, Vofopitant, Ezlopitant, Lanepitant. Also similar results are observed with treatment with NK1 receptor antibodies.

**Table 37. Percentage of inhibition of cell cultures treated with the antagonist Aprepitant in relation to the presence of a constitutively activated form of NK1 receptor (Group 1) or the presence of a normal form of the receptor (Group II).**

| **Tumor type (histologic type)** | **Control** | **Group I** | **Group II** |
|---|---|---|---|
| Gastric carcinoma (Adenocarcinoma) | 100 | 76±6 | 95±7 |
| Colon carcinoma (Adenocarcinoma) | 100 | 75±4 | 96±6 |
| Pancreatic carcinoma (Adenocarcinoma) | 100 | 76±7 | 96±4 |
| Renal cell carcinoma (clear cell renal cell carcinoma) | 100 | 76±6 | 97±6 |
| Breast carcinoma (ductal adenocarcinoma) | 100 | 75±6 | 96±4 |
| Ovarian carcinoma (Adenocarcinoma) | 100 | 77±7 | 97±5 |
| Endometrium carcinoma | 100 | 78±6 | 95±3 |
| Carcinoma of the uterine cervix (Squamous cell carcinoma). | 100 | 77±7 | 94±4 |
| Lung carcinoma (small cell lung carcinoma) | 100 | 78±5 | 93±3 |
| Lung carcinoma (non small cell lung carcinoma) | 100 | 73±7 | 95±5 |
| Thyroid carcinoma (follicular carcinoma). | 100 | 73±4 | 94±3 |
| Thyroid carcinoma(papillary carcinoma). | 100 | 75±6 | 95±3 |
| Urinary bladder carcinoma (transitional cell carcinoma) | 100 | 76±5 | 93±4 |
| Prostate carcinoma | 100 | 77±6 | 95±5 |
| Glioma (astrocytoma) | 100 | 73±7 | 96±3 |
| Undifferentiated Pleomorphic Sarcoma (malignant fibrous histiocytoma grade 3). | 100 | 75±6 | 92±5 |
| Melanoma (Nodular Melanoma). | 100 | 74±4 | 94±4 |
| Non Hodgkin Lymphoma B (diffuse B cell lymphoma). | 100 | 74±6 | 93±3 |
| Non Hodgkin Lymphoma T (diffuse T cell lymphoma). | 100 | 75±6 | 96±4 |
| Burkitt Lymphoma | 100 | 74±6 | 97±5 |

## Claims

1. An *in vitro* method to predict the response of a human subject suffering from cancer to a treatment of said disease with an antagonist of the NK1 receptor, wherein said method comprises:
- detecting the expression level of the NK1 receptor in a sample of tumor cells isolated from the subject;.
- comparing the expression level to a reference value; and
- predicting a positive response to the treatment if the expression level is increased relative to the reference value,
wherein the reference value is the average expression level in a population of non-respondents.

2. The *in vitro* method of claim 1, wherein the expression level of the NK1 receptor is determined by detecting the level of the NK1 receptor or of the mRNA encoding the same.

3. The *in vitro* method according to claim 1 or 2, wherein the expression of the NK1 receptor is defined as increased if the level of expression of the NK1 receptor is increased in more than 20% of the cells of the isolated biological sample.

4. The *in vitro* method according to any claims 1 to 3 wherein the presence or the absence of mutations in the K-ras gene in the tumor cells of the isolated sample is also determined and wherein the absence of mutation in the K-ras gene is indicative of a positive response to the treatment.

5. The *in vitro* method according to any of the claims 1 to 4, wherein the presence or the absence of the ERK, and/or MEK, and/or AKT, and/or the truncated form of the NK1 receptor or any combinations thereof is also determined in the tumor cells of the isolated sample and wherein the presence of any of the proteins ERK, MEK, AKT, the truncated form of the NK1 receptor, or any combination thereof, or the presence of the mRNA encoding any of those proteins, is indicative of a positive response to the treatment.

6. The *in vitro* method according to any of claims 1 to 5, wherein the presence or absence of a constitutively activated form of the NK1 receptor in the tumor cells of the isolated sample is also determined and wherein the presence of a constitutively activated form of the NK1 receptor is indicative of a positive response to the treatment.

7. The *in vitro* method according to any of the previous claims, wherein the detection
a. of the expression level of NK1 receptor
b. the presence or absence of mutations in the *K-ras* gene,
c. the presence of the ERK protein, or the presence of the mRNA encoding the same,
d. the presence of the MEK protein, or the presence of the mRNA encoding the same,
e. the presence of the AKT protein, or the mRNA encoding the same, or
f. the presence or absence of the truncated form of the NK1 receptor, is executed by means of
i. a procedure having a genetic profile, and/or
ii. a procedure encompassing PCR; and/or
iii. Northern blot transference, and/or
iv. an immunohistochemical procedure.

8. The *in vitro* method according to any of claims 1 to 7, wherein said human subject has not been previously treated with an antagonist of the NK1 receptor.

9. The *in vitro* method according to any of claims 1 to 8, wherein the type of cancer is selected among the following: gastric carcinoma, gastric adenocarcinoma, colon carcinoma, colon adenocarcinoma, pancreatic carcinoma, pancreatic adenocarcinoma, renal cell carcinoma, clear cell renal cell carcinoma, breast carcinoma, breast adenocarcinoma, ovarian carcinoma, ovarian adenocarcinoma, endometrial carcinoma, carcinoma of the uterine cervix, lung carcinoma, non-small-cell lung cancer and/or small-cell lung cancer, thyroid carcinoma, papillary thyroid carcinoma and/or follicular thyroid carcinoma, bladder carcinoma, transitional cell carcinoma of the urinary bladder, prostate carcinoma, cancer of glial lineage of the central nervous system (glioma), sarcomas, fibrosarcoma, malignant fibrous histiocytoma and Edwing sarcoma, melanoma, embryonal cancers, neuroblastoma; and hematological cancers, B-cell or T-cell leukemia, non-Hodgkin lymphoma, non Hodgkin lymphoma B-cell or T-cell types, Burkitt lymphoma, Hodgkin lymphoma and multiple myeloma.

10. The *in vitro* method according to any of claims 1 to 9, wherein the antagonists of the NKl receptor are non-peptidic or peptidic antagonists.

11. The *in vitro* method according to claim 10, wherein the non-peptidic antagonists are selected among: Aprepitant, Vestipitant, Casopitant, Vofopitant, Ezlopitant, Lanepitant, LY-686017, L-733,060, L-732,138, L-703,606, WIN 62,577, TAK-637, YR673, CP-100263, WIN 51708, CP-96345, L-760735, CP-122721, L-758298, L-741671, L-742694, CP-99994, T-2328, or combinations thereof; and the peptidic antagonists are selected among antibodies or fragments of them, directed against the NK1 receptor.

12. Use of the expression level of the NK1 receptor in a sample of tumor cells as an indicator in an *in vitro* method to predict if a human suffering from cancer will have a positive response to treatment with a pharmaceutical composition comprising an antagonist of the NK1 receptor.

13. Use according to claim 12, wherein the antagonist of the NK1 receptor is a non-peptidic or peptidic antagonist.

14. Use according to claim 13, wherein the non-peptidic antagonist is selected from: Aprepitant, Vestipitant, Casopitant, Vofopitant, Ezlopitant, Lanepitant, LY-686017, L-733,060, L-732,138, L-703,606, WIN 62,577, TAK637, YR673, CP-100263, WIN 51708, CP-96345, L-760735, CP-122721, L-758298, L-741671, L-742694, CP-99994, T-2328, or combinations thereof; and the peptidic antagonist is selected from antibodies or fragments of thereof directed against the NK1 receptor.

## Patentansprüche

1. *In*-*vitro*-Verfahren, um das Ansprechen eines menschlichen Subjekts, das an Krebs leidet, auf eine Behandlung der Erkrankung mit einem Antagonisten des NK1-Rezeptors vorherzusagen, wobei das Verfahren Folgendes umfasst:
- Nachweisen des Expressionsniveaus des NK1-Rezeptors in einer Probe von Tumorzellen, die aus dem Subjekt isoliert wurde;
- Vergleichen des Expressionsniveaus mit einem Referenzwert; und
- Vorhersagen eines positiven Ansprechens auf die Behandlung, wenn das Expressionsniveau relativ zum Referenzwert erhöht ist,
wobei der Referenzwert das durchschnittliche Expressionsniveau in einer Population von nicht-ansprechenden Individuen ist.

2. *In*-*vitro*-Verfahren nach Anspruch 1, wobei das Expressionsniveau des NK1-Rezeptors durch Nachweisen des Niveaus des NK1-Rezeptors oder der mRNA, die denselben codiert, bestimmt wird.

3. *In*-*vitro*-Verfahren nach Anspruch 1 oder 2, wobei die Expression des NK1-Rezeptors als erhöht definiert wird, wenn das Niveau der Expression des NK1-Rezeptors in mehr als 20 % der Zellen der isolierten biologischen Probe erhöht ist.

4. *In-vitro*-Verfahren nach einem der Ansprüche 1 bis 3, wobei die Anwesenheit oder die Abwesenheit von Mutationen in dem K-Ras-Gen in den Tumorzellen der isolierten Probe ebenfalls bestimmt wird und wobei die Abwesenheit von Mutation in dem K-Ras-Gen auf ein positives Ansprechen auf die Behandlung hindeutet.

5. *In*-*vitro*-Verfahren nach einem der Ansprüche 1 bis 4, wobei die Anwesenheit oder die Abwesenheit des ERK und/oder MEK und/oder AKT und/oder der gekürzten Form des NK1-Rezeptors oder von Kombinationen davon ebenfalls in den Tumorzellen der isolierten Probe bestimmt wird und wobei die Anwesenheit eines der Proteine ERK, MEK, AKT, der gekürzten Form des NK1-Rezeptors oder einer Kombination davon oder die Anwesenheit der mRNA, die eines dieser Proteine codiert, auf ein positives Ansprechen auf die Behandlung hindeutet.

6. *In-vitro*-Verfahren nach einem der Ansprüche 1 bis 5, wobei die Anwesenheit oder Abwesenheit einer konstitutiv aktivierten Form des NK1-Rezeptors in den Tumorzellen der isolierten Probe ebenfalls bestimmt wird und wobei die Anwesenheit einer konstitutiv aktivierten Form des NK1-Rezeptors auf ein positives Ansprechen auf die Behandlung hindeutet.

7. *In-vitro*-Verfahren nach einem der vorigen Ansprüche, wobei der Nachweis
a. des Expressionsniveaus von NK1-Rezeptor,
b. der Anwesenheit oder Abwesenheit von Mutationen in dem *K-Ras*-Gen*,*
c. der Anwesenheit des ERK-Proteins oder der Anwesenheit der mRNA, die dasselbe codiert,
d. der Anwesenheit des MEK-Proteins oder der Anwesenheit der mRNA, die dasselbe codiert,
e. der Anwesenheit des AKT-Proteins oder der mRNA, die dasselbe codiert, oder
f. der Anwesenheit oder Abwesenheit der gekürzten Form des NK1-Rezeptors mittels Folgendem ausgeführt wird:
i. einer Prozedur mit einem genetischen Profil und/oder
ii. einer Prozedur, die PCR einschließt; und/oder
iii. Northern-Blot-Übertragung und/oder
iv. einer immunhistochemischen Prozedur.

8. *In-vitro*-Verfahren nach einem der Ansprüche 1 bis 7, wobei das menschliche Subjekt zuvor nicht mit einem Antagonisten des NK1-Rezeptors behandelt wurde.

9. *In-vitro*-Verfahren nach einem der Ansprüche 1 bis 8, wobei der Typ des Krebses unter den folgenden ausgewählt ist: Magenkarzinom, Magenadenokarzinom, Darmkarzinom, Darmadenokarzinom, Pankreaskarzinom, Pankreasadenokarzinom, Nierenzellkarzinom, klarzelliges Nierenzellkarzinom, Brustkarzinom, Brustadenokarzinom, Eierstockkarzinom, Eierstockadenokarzinom, Endometriumkarzinom, Karzinom des Gebärmutterhalses, Lungenkarzinom, nichtkleinzelliger Lungenkrebs und/oder kleinzelliger Lungenkrebs, Schilddrüsenkarzinom, papilläres Schilddrüsenkarzinom und/oder follikuläres Schilddrüsenkarzinom, Blasenkarzinom, Übergangszellkarzinom, Karzinom der Harnblase, Prostatakarzinom, Krebs der Gliazelllinie des zentralen Nervensystems (Gliom), Sarkom, Fibrosarkom, malignes fibröses Histiozytom und Edwing-Sarkom, Melanom, embryonale Krebse, Neuroblastom; und hämatologische Krebse, B-Zell- oder T-Zell-Leukämie, Non-Hodgkin-Lymphom, Non-Hodgkin-Lymphom vom B-Zell- oder T-Zelltyp, Burkitt-Lymphom, Hodgkin-Lymphom und multiples Myelom.

10. *In-vitro*-Verfahren nach einem der Ansprüche 1 bis 9, wobei die Antagonisten des NK1-Rezeptors nicht-peptidische oder peptidische Antagonisten sind.

11. *In-vitro*-Verfahren nach Anspruch 10, wobei die nicht-peptidischen Antagonisten ausgewählt sind unter: Aprepitant, Vestipitant, Casopitant, Vofopitant, Ezlopitant, Lanepitant, LY-686017, L-733,060, L-732,138, L-703,606, WIN 62,577, TAK-637, YR673, CP-100263, WIN 51708, CP-96345, L-760735, CP-122721, L-758298, L-741671, L-742694, CP-99994, T-2328 oder Kombinationen davon; und die peptidischen Antagonisten unter Antikörpern oder Fragmenten dieser, die gegen den NK1-Rezeptor gerichtet sind, ausgewählt sind.

12. Verwendung des Expressionsniveaus des NK1-Rezeptors in einer Probe von Tumorzellen als einen Indikator in einem In-vitro-Verfahren, um vorherzusagen, ob ein Mensch, der an Krebs leidet, positiv auf Behandlung mit einer pharmazeutischen Zusammensetzung, umfassend einen Antagonisten des NK1-Rezeptors, ansprechen wird.

13. Verwendung nach Anspruch 12, wobei der Antagonist des NK1-Rezeptors ein nicht-peptidischer oder peptidischer Antagonist ist.

14. Verwendung nach Anspruch 13, wobei der nicht-peptidische Antagonist ausgewählt ist aus: Aprepitant, Vestipitant, Casopitant, Vofopitant, Ezlopitant, Lanepitant, LY-686017, L-733,060, L-732,138, L-703,606, WIN 62,577, TAK637, YR673, CP-100263, WIN 51708, CP-96345, L-760735, CP-122721, L-758298, L-741671, L-742694, CP-99994, T-2328 oder Kombinationen davon; und der peptidische Antagonist aus Antikörpern oder Fragmenten davon, die gegen den NK1-Rezeptor gerichtet sind, ausgewählt ist.

## Revendications

1. Procédé *in vitro* pour prédire la réponse d'un sujet humain atteint de cancer à un traitement de ladite maladie avec un antagoniste du récepteur NK1, ledit procédé comprenant :
- la détection du taux d'expression du récepteur NK1 dans un échantillon de cellules tumorales isolé à partir du sujet ;
- la comparaison du taux d'expression à une valeur de référence ; et
- la prédiction d'une réponse positive au traitement si le taux d'expression est accru par rapport à la valeur de référence,
la valeur de référence étant le taux d'expression moyen au sein d'une population de non-répondants.

2. Procédé *in vitro* selon la revendication 1, dans lequel le taux d'expression du récepteur NK1 est déterminé par détection du taux du récepteur NK1 ou de l'ARNm codant pour celui-ci.

3. Procédé *in vitro* selon la revendication 1 ou 2, dans lequel le taux d'expression du récepteur NK1 est défini comme accru si le taux d'expression du récepteur NK1 est accru dans plus de 20 % des cellules de l'échantillon biologique isolé.

4. Procédé *in vitro* selon l'une quelconque des revendications 1 à 3, dans lequel la présence ou l'absence de mutations dans le gène K-ras dans les cellules tumorales de l'échantillon isolé est également déterminée et dans lequel l'absence de mutation dans le gène K-ras indique une réponse positive au traitement.

5. Procédé *in vitro* selon l'une quelconque des revendications 1 à 4, dans lequel la présence ou l'absence de ERK, et/ou MEK, et/ou AKT, et/ou de la forme tronquée du récepteur NK1 ou de toute combinaison de celles-ci est également déterminée dans les cellules tumorales de l'échantillon isolé et dans lequel la présence de l'une quelconque des protéines ERK, MEK, AKT, de la forme tronquée du récepteur NK1, ou de toute combinaison de celles-ci, ou la présence de l'ARNm codant pour l'une quelconque de ces protéines, indique une réponse positive au traitement.

6. Procédé *in vitro* selon l'une quelconque des revendications 1 à 5, dans lequel la présence ou l'absence d'une forme constitutivement active du récepteur NK1 dans les cellules tumorales de l'échantillon isolé est également déterminée et dans lequel la présence d'une forme constitutivement active du récepteur NK1 indique une réponse positive au traitement.

7. Procédé *in vitro* selon l'une quelconque des revendications précédentes, dans lequel la détection
a. du taux d'expression du récepteur NK1,
b. de la présence ou de l'absence de mutations dans le gène *K-ras*,
c. de la présence de la protéine ERK, ou de la présence de l'ARNm codant pour celle-ci,
d. de la présence de la protéine MEK, ou de la présence de l'ARNm codant pour celle-ci,
e. de la présence de la protéine AKT, ou de l'ARNm codant pour celle-ci, ou
f. de la présence ou de l'absence de la forme tronquée du récepteur NK1, est exécutée au moyen
i. d'une procédure ayant un profil génétique, et/ou
ii. d'une procédure englobant une PCR ; et/ou
iii. d'un transfert de Northern, et/ou
iv. d'une procédure immunohistochimique.

8. Procédé *in vitro* selon l'une quelconque des revendications 1 à 7, dans lequel ledit sujet humain n'a pas été précédemment traité avec un antagoniste du récepteur NK1.

9. Procédé *in vitro* selon l'une quelconque des revendications 1 à 8, dans lequel le type de cancer est chois parmi les suivants : carcinome gastrique, adénocarcinome gastrique, carcinome du côlon, adénocarcinome du côlon, carcinome pancréatique, adénocarcinome pancréatique, carcinome des cellules rénales, carcinome des cellules rénales à cellules claires, carcinome du sein, adénocarcinome du sein, carcinome ovarien, adénocarcinome ovarien, carcinome endométrial, carcinome du col utérin, carcinome du poumon, cancer du poumon non à petites cellules et/ou cancer du poumon à petites cellules, carcinome de la thyroïde, carcinome papillaire de la thyroïde et/ou carcinome folliculaire de la thyroïde, carcinome de la vessie, carcinome à cellules transitionnelles de la vessie, carcinome de la prostate, cancer de lignées gliales du système nerveux central (gliome), sarcomes, fibrosarcome, histiocytome fibreux malin et sarcome d'Edwing, mélanome, cancers embryonnaires, neuroblastome ; et cancers hématologiques, leucémie des cellules B ou des cellules T, lymphome non hodgkinien, lymphome non hodgkinien de type à cellules B ou à cellules T, lymphome de Burkitt, lymphome hodgkinien et myélome multiple.

10. Procédé *in vitro* selon l'une quelconque des revendications 1 à 9, dans lequel les antagonistes du récepteur NK1 sont des antagonistes non peptidiques ou peptidiques.

11. Procédé *in vitro* selon la revendication 10, dans lequel les antagonistes non peptidiques sont choisis parmi : aprépitant, vestipitant, casopitant, vofopitant, ezlopitant, lanépitant, LY-686017, L-733,060, L-732,138, L-703,606, WIN 62,577, TAK-637, YR673, CP-100263, WIN 51708, CP-96345, L-760735, CP-122721, L-758298, L-741671, L-742694, CP-99994, T-2328, ou des combinaisons de ceux-ci, et les antagonistes peptidiques sont choisis parmi des anticorps ou fragments de ceux-ci, dirigés contre le récepteur NK1.

12. Utilisation du taux d'expression du récepteur NK1 dans un échantillon de cellules tumorales en tant qu'indicateur dans un procédé *in vitro* pour prédire si un être humain atteint de cancer présentera une réponse positive au traitement avec une composition pharmaceutique comprenant un antagoniste du récepteur NK1.

13. Utilisation selon la revendication 12, dans laquelle l'antagoniste du récepteur NK1 est un antagoniste non peptidique ou peptidique.

14. Utilisation selon la revendication 13, dans laquelle l'antagoniste non peptidique est choisi parmi : aprépitant, vestipitant, casopitant, vofopitant, ezlopitant, lanépitant, LY-686017, L-733,060, L-732,138, L-703,606, WIN 62,577, TAK-637, YR673, CP-100263, WIN 51708, CP-96345, L-760735, CP-122721, L-758298, L-741671, L-742694, CP-99994, T-2328, ou des combinaisons de ceux-ci ; et l'antagoniste peptidique est choisi parmi des anticorps ou fragments de ceux-ci dirigés contre le récepteur NK1.
